# EUROPEAN PATENT SPECIFICATION

(11) **EP 1 693 358 B1**
(45) Date of publication and mention of the grant of the patent: **08.09.2010**
(21) Application number: 04819487.2
(22) Date of filing: 26.11.2004
(51) Int. Cl.: C07C 49/84, C07D 249/08, C07D 303/22, C07D 405/06

(54) **PROCESS FOR PRODUCING EPOXYTRIAZOLE COMPOUND AND INTERMEDIATE THEREFOR**
VERFAHREN ZUR HERSTELLUNG EINER EPOXYTRIAZOLVERBINDUNG UND ZWISCHENPRODUKT DAFÜR
PROCESSUS DE PRODUCTION DE COMPOSE EPOXYTRIAZOLE ET INTERMEDIAIRE A CET EFFET

(30) Priority: 27.11.2003 JP 2003398252
(43) Date of publication of application: 23.08.2006
(73) Proprietor: SUMITOMO CHEMICAL CO., LTD., Tokyo 104-8260 (JP)
(72) Inventor: WANG, Weiqi, Ibaraki-shi, Osaka 5670048 (JP); IKEMOTO, Tetsuya, Toyonaka-shi, Osaka 5610874 (JP)
(74) Representative: Duckworth, Timothy John
(86) International application number: PCT/JP2004/017992
(87) International publication number: WO 2005/051879

(56) References cited:
- WO-A1-99/45008
- WO-A1-03/068758
- JP-A- 4 074 168
- JP-A- 5 230 038
- KONOSU T. ET AL: 'Triazole Antifungals. III. Stereocontrolled Synthesis of an Optically Active Triazolylmethyloxirane Precursor to Antifungal Oxazolidine Derivatives' CHEM.PHARM.BULL. vol. 39, no. 9, 1991, pages 2241 - 2246, XP002108703

## Description

### TECHNICAL FIELD

The present invention relates to a process for producing epoxytriazole compounds useful as synthetic intermediates of antifungal agents.

### BACKGROUND ART

An epoxytriazole compound represented by the formula (VII) (hereinafter, also referred to as an epoxytriazole compound (VII)): 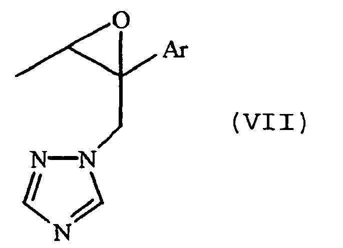 wherein Ar represents a difluorophenyl group,
is a useful compound which is suited for use as a synthetic intermediate of an antifungal agent (see, for example JP H04-356471-A).

Various processes for producing an epoxytriazole compound (VII) have hitherto been proposed (see, for example, JP H04-356471-A, EP 421600-A, JP H05-230038-A, "Chemical & Pharmaceutical Bulletin", The Pharmaceutical Society of Japan, 1993, Vol. 41, No. 6, p.1035-1042, etc.) and they are processes using a compound represented by the formula (1) (hereinafter, also referred to as the compound (1)) in which a hydroxyl group is protected with a tetrahydropyranyl group, as a raw material, as shown in the following scheme: 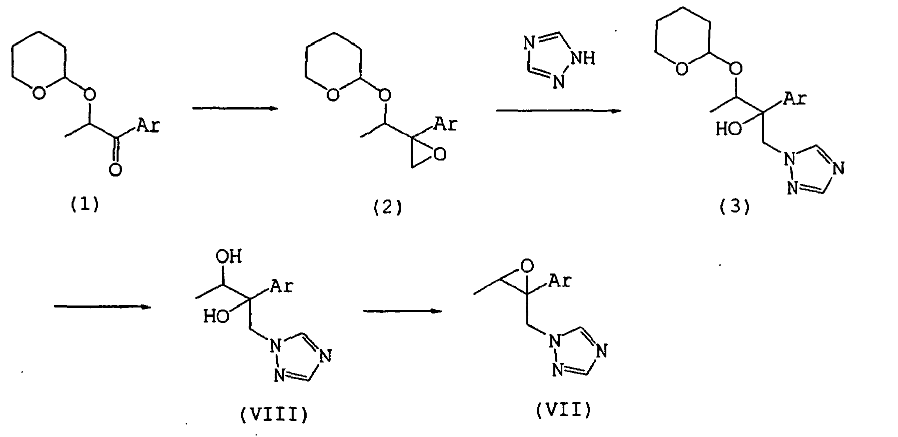 wherein Ar represents a difluorophenyl group.

However, the compound (1) as a common raw material for these production processes is hardly purified by recrystallization because it is in liquid state at normal temperature and is a mixture of two diastereomers. Furthermore, it is difficult to purify by distillation because a tetrahydropyranyl group, as a protective group, is easily eliminated by heat.

Pharmaceutical products are obligated to be produced under severe quality control in accordance with Good Manufacturing Practice (hereinafter abbreviated to GMP) so as to secure safety. However, as described above, the compound (1) as a starting material of a conventional method is hardly purified from an industrial point of view, and thus it is difficult to control the purity thereof. Therefore, there was a problem of quality control in the production of the epoxytriazole compound (VII) which is an important synthetic intermediate in accordance with GMP.

An object of the present invention is to provide a novel process capable of producing an epoxytriazole compound (VII) as a synthetic intermediate of a pharmaceutical product under proper quality control.

Another object of the present invention is to provide an intermediate which is useful in such a production process.

These objects and other objects will become apparent from the following descriptions.

### DISCLOSURE OF THE INVENTION

The present inventors have studied intensively studied about protective groups to be replaced by the tetrahydropyranyl group of the compound (1) so as to accomplish the above objects, and thus the present invention has been completed.

That is, the present invention is directed to the followings:
<1> A compound of the formula (I); 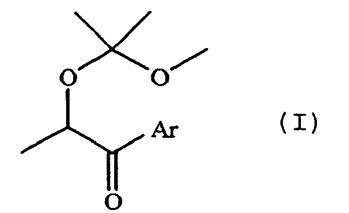 wherein Ar represents difluorophenyl group.
<2> The compound according to <1>, wherein Ar is 2,4-difluorophenyl group.
<3> The compound according to <1> or <2>, which has a steric configuration of the formula ( Ia ); 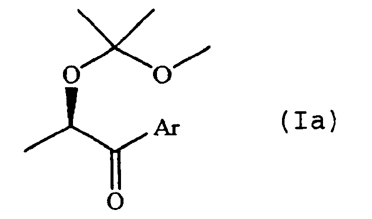 wherein Ar has the same meaning as defined above.
<4> A compound of the formula (II); 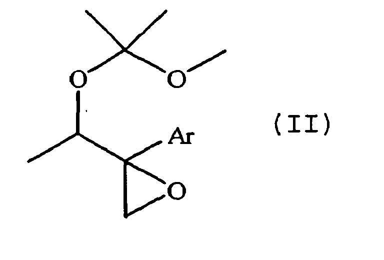 wherein Ar represents difluorophenyl group.
<5> The compound according to <4>, wherein Ar is 2,4-difluorophenyl group.
<6> The compound according to <4> or <5>, which has a steric configuration of the formula ( IIa ); 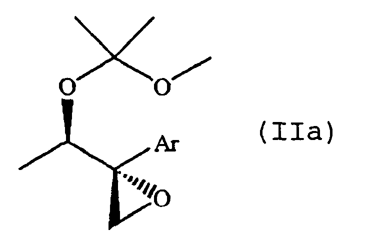 wherein Ar has the same meaning as defined above.
<7> A compound of the formula (III) or a salt thereof; 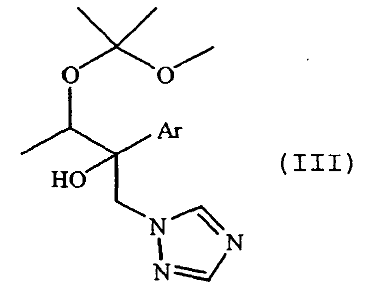 wherein Ar has the same meaning as defined above.
<8> The compound or a salt thereof according to <7>, wherein Ar is 2,4-difluorophenyl group.
<9> The compound or a salt thereof according to <7>, which has a steric configuration of the formula ( IIIa); 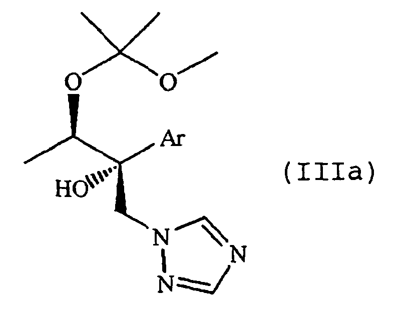 wherein Ar has the same meaning as defined above.
<10> A process for producing a compound of the formula (VII) or a salt thereof; 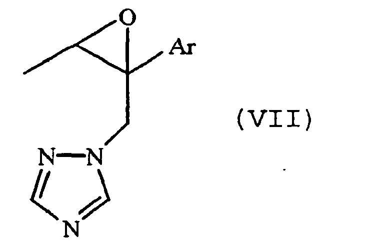 wherein Ar represents difluorophenyl group,
   which comprises the following Steps (a), (b), (c) and (d). Step (a): a step for epoxydizing a compound of the formula (I) 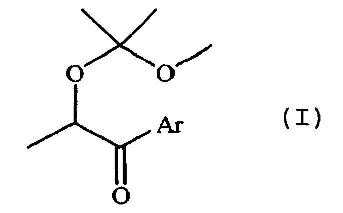 wherein Ar has the same meaning as defined above,
   to obtain a compound of the formula (II); 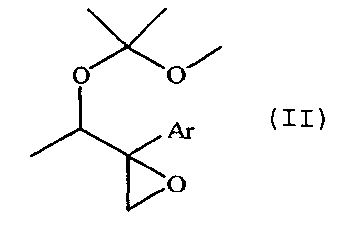 wherein Ar has the same meaning as defined above
   Step (b): a step for deprotecting the compound of the formula (II) to obtain a compound of the formula (IV); 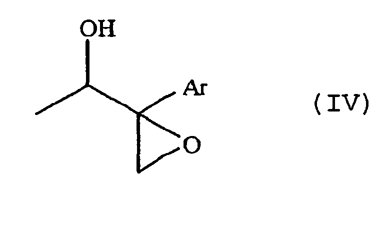 wherein Ar has the same meaning as defined above
   Step (c): a step for reacting the compound of the formula (IV) with a compound of the formula (V)

   RSO₂X (V)

   wherein R represents lower alkyl group, phenyl group or tolyl group, and X represents halogen atom,
   to obtain a compound of the formula (VI); 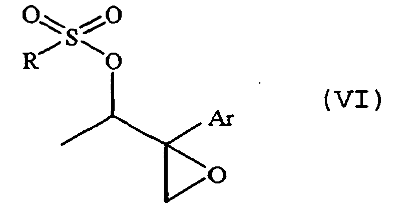 wherein R and X have the same meanings as defined above
   Step (d): a step for reacting the compound of the formula (VI) with 1,2,4-triazole to obtain the compound of the formula (VII)
<11> A process for producing a compound of the formula (VII) or a salt thereof; 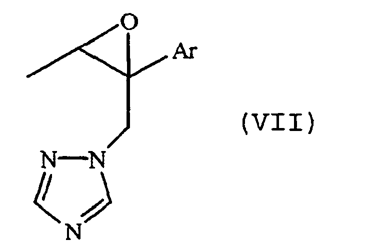 wherein Ar represents difluorophenyl group,
   which comprises the following Steps (a), (e), (f), (g) and (h).
   Step (a): a step for epoxydizing a compound of the formula (I); 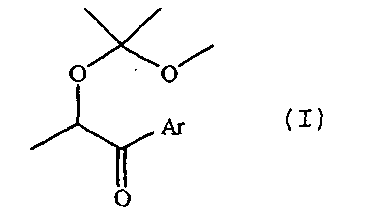 wherein Ar has the same meaning as defined above,
   to obtain a compound of the formula (II); 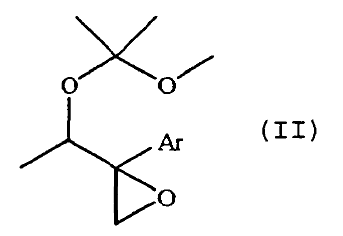 wherein Ar has the same meaning as defined above
   Step (e): a step for reacting the compound of the formula (II) with 1,2,4-triazole to obtain a compound of the formula (III) or a salt thereof; 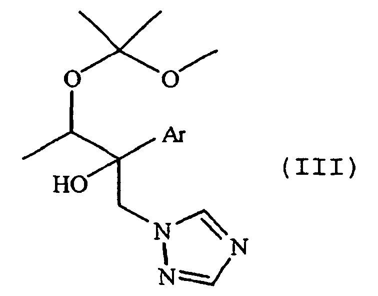 wherein Ar has the same meaning as defined above
   Step (f): a step for deprotecting the compound of the formula (III) or the salt thereof to obtain a compound of the formula (VIII) or a salt thereof; 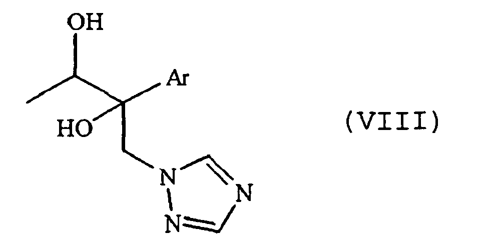 wherein Ar has the same meaning as defined above
   Step (g): a step for reacting the compound of the formula (VIII) or the salt thereof with a compound of the formula (V);

   RSO₂X (V)

   wherein R represents an alkyl group having 1 to 12 carbon atoms, phenyl group or tolyl group, and X represents halogen atom,
   to obtain a compound of the formula (IX); 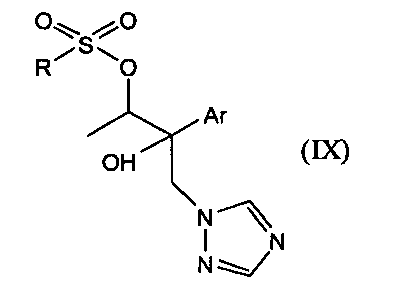 wherein R and X have the same meanings as defined above
   Step (h): a step for treating the compound of the formula (IX) or a salt thereof with a base to obtain the compound of the formula (VII) or a salt thereof.
<12> A process for producing a compound of the formula (I); 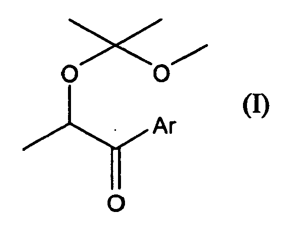 wherein Ar represents difluorophenyl group, which comprises a step for reacting a compound of the formula (XI);

   ArX² (XI)

   wherein Ar represents difluorophenyl group and X² represents halogen atom, with a Grignard reagent of the formula (X);

   R¹MgX¹ (X)

   wherein R¹ represents hydrocarbon group and X¹ represents halogen atom, to obtain an arylmagnesium halide of the formula (XII);

   ArMgX¹ (XII)

   wherein Ar and X¹ have the same meaning as defined above,
   and a step for reacting a compound of the formula (XIII'); 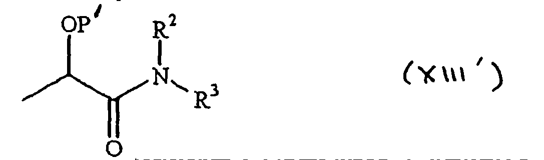 wherein P, represents a (1-methoxy-1-methyl)ethyl group, and R² and R³ each independently represent an alkyl group having 1 to 12 carbon atoms or an alkoxy group having 1 to 8 carbon atoms or R² and R³ bond to form divalent acyclic hydrocarbon group which forms aliphatic heterocyclic ring together with an adjacent nitrogen atom, and one of -CH₂- in the divalent acyclic hydrocarbon group may be substituted by -O-, with the proviso that R² and R³ don't represent alkoxy groups having 1 to 8 carbon atoms simultaneously,
   with the compound of the arylmagnesium halide of the formula (XII).
<13> A process for producing a compound of the formula (I); 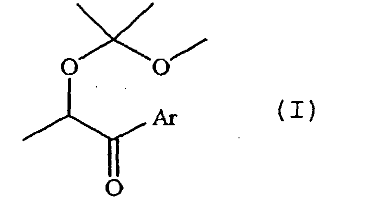 wherein Ar represents difluorophenyl group,
   which comprises the following Steps (i), (j),(k) and (1).
   Step (i): a step for reacting a compound of the formula (XI);

   ArX² (XI)

   wherein Ar represents difluorophenyl group and X² represents halogen atom, with a Grignard reagent of the formula (X);

   R¹MgX¹ (X)

   wherein R¹ represents hydrocarbon group and X¹ represents halogen atom, to obtain an arylmagnesium halide of the formula (XII);

   ArMgX¹ (XII)

   wherein Ar and X¹ have the same meaning as defined above
   Step (j): a step for reacting a compound of the formula (XIII); 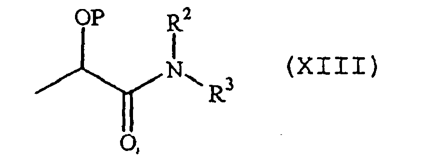 wherein P is a protecting group for a hydroxyl group, and R² and R³ each independently represent an alkyl group having 1 to 12 carbon atoms or an alkoxy group having 1 to 8 carbon atoms or R² and R³ bond to form divalent acyclic hydrocarbon group which forms aliphatic heterocyclic ring together with an adjacent nitrogen atom, and one of -CH₂- in the divalent acyclic hydrocarbon group may be substituted by -O-, with the proviso that R² and R³ don't represent alkoxy groups having 1 to 8 carbon atoms simultaneously,
   with the compound of the arylmagnesium halide of the formula (XII) to obtain a compound of the formula (XIV); 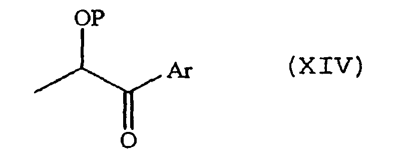 wherein P and Ar have the same meanings as defined above
   Step (k): a step for deprotecting the compound of the formula (XIV) to obtain a compound of the formula (XV); 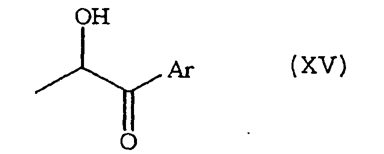 wherein Ar has the same meaning as defined above
   Step (1): a step for reacting the compound of the formula (XV) with 2-methoxypropene to obtain the compound of the formula (I)

### BEST MODE FOR CARRYING OUT THE INVENTION

The present invention will now be described in detail.

First, the respective symbols used in the present specification will be described.

Ar represents difluorophenyl group of hydrogen atom, and examples thereof include 2,3-difluorophenyl group, 2,4-difluorophenyl group, 2,5-difluorophenyl group, 2,6-difluorophenyl group, 3,4-difluorophenyl group, 3,5-difluorophenyl group, and the like, and 2,4-difluorophenyl group and 2,5-difluorophenyl group are preferred.

R represents an alkyl group having 1 to 12 carbon atoms, phenyl group or tolyl group.

R may be a linear or branched alkyl group having 1 to 12 carbon atoms, and more preferably 1 to 3 carbon atoms. Examples thereof include methyl, ethyl, propyl, isopropyl, butyl, isobutyl, sec-butyl, tert-butyl, pentyl, isopentyl, neopentyl, hexyl, heptyl, octyl, nonyl, decyl, undecyl, dodecyl, and the like, and methyl is preferable.

Tolyl group on R may be any of p-tolyl group, m-tolyl group and o-tolyl group, and preferably p-tolyl group.

X represents halogen atom. Examples of the halogen atom include chlorine atom, bromine atom, iodine atom, and the like, and chlorine atom and bromine atom are preferable.

When compounds (I), (II), (III), (IV), (VI), (VIII) and (IX), and the epoxytriazole compound (VII) have at least one asymmetric carbon atom, these compounds may be any one of optically active isomers or mixture thereof (for example, racemic mixture, enantiomeric mixture, diastereomeric mixture, etc.). Compounds having preferable configuration are compounds represented by the following formulas (Ia), (IIa), (IIIa), (IVa), (VIa), (VIIa), (VIIIa) and (IXa) or enantiomers thereof: 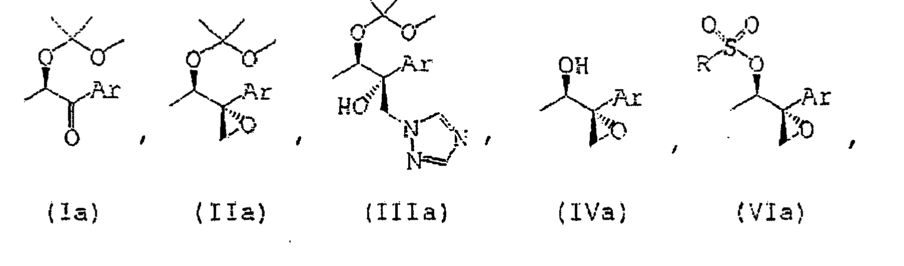 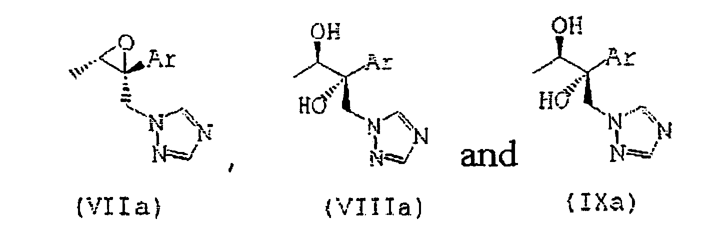 wherein R and Ar are as defined above.

The compounds (III), (VIII) and (IX) and the epoxytriazole compound (VII) have a 1,2,4-triazole ring and may be in the form of salt. Examples of the salts include salts with inorganic acids (for example, hydrochloric acid, sulfuric acid, nitric acid, phosphoric acid, etc.) and with organic acids (for example, acetic acid, propionic acid, methanesulfonic acid, 4-toluenesulfonic acid, etc.).

The production process according to the present invention is shown by the following scheme:

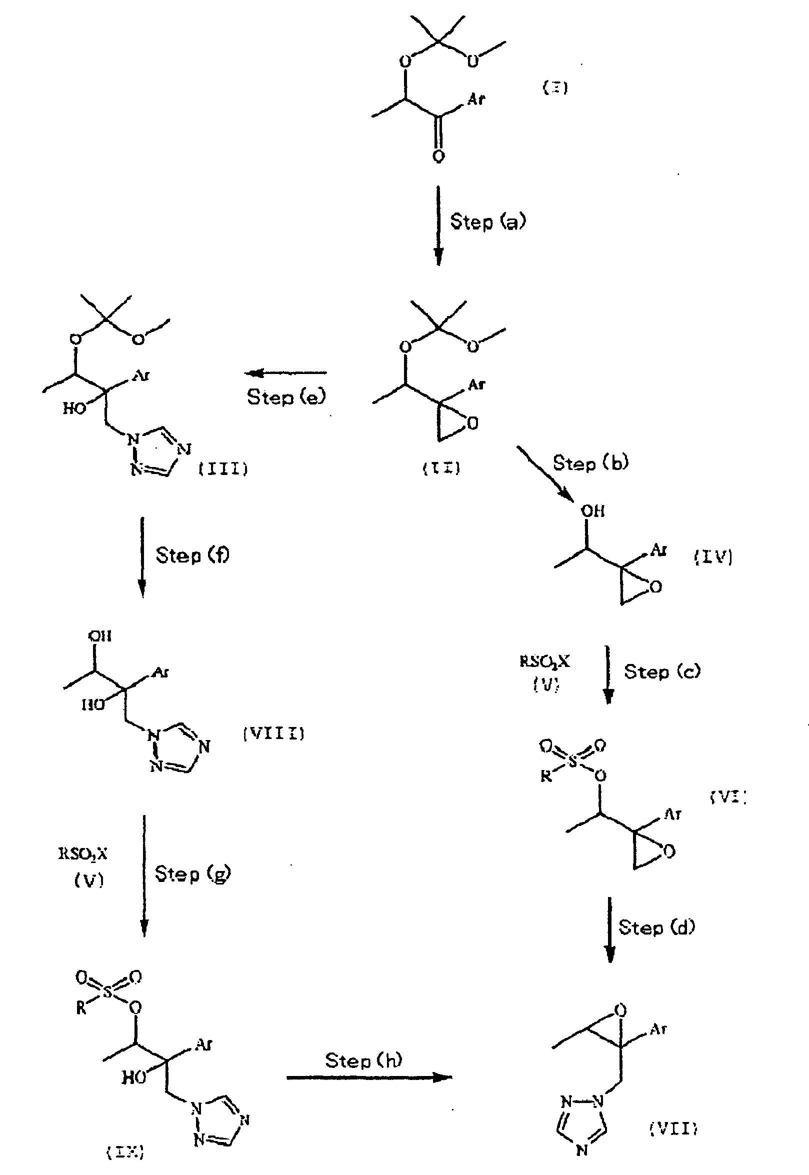

That is, the process of the present invention is a process for producing the epoxytriazole compound (VII), comprising Steps (a), (b), (c) and (d), or comprising Steps (a), (e), (f), (g) and (h). In the present invention, it is possible to purify the compound (I), as a starting material, by recrystallization in any route and to easily control the purity of the raw material in the production of the epoxytriazole compound (VII), and therefore quality control in accordance with GMP can be advantageously conducted. The respective steps will now be described.

### 1. Step (a)

Step (a) is the step for obtaining the compound (II) by epoxidizing the compound (I). For example, the compound (II) can be obtained by reacting the compound (I) with trimethyloxosulfonium salt or trimethylsulfonium salt in the presence of a base in a solvent. The order of the addition of reagents is not specifically limited. For example, there may be employed a method comprising the steps of charging trimethyloxosulfonium salt or trimethylsulfonium salt and a base in a solvent in a reaction vessel and adding the compound (I) thereto, or a method comprising the steps of charging trimethyloxosulfonium salt or trimethylsulfonium salt in a solvent in a reaction vessel, adding a base thereto to react them and adding the reaction solution to a solution obtained by dissolving the compound (I) in a solvent.

The base used in Step (a) is not limited as long as it reacts with trimethyloxosulfonium salt or trimethylsulfonium salt to produce sulfur ylide, and examples thereof include alkali metal hydroxides such as potassium hydroxide, sodium hydroxide, lithium hydroxide, and the like; alkali metal hydrides such as sodium hydride, potassium hydride, lithium hydride, and the like; alkyl alkali metals such as n-butyl lithium, methyl lithium, n-hexyl lithium, and the like; alkali metal amides such as sodium amide, potassium amide, lithium diisopropylamide, lithium dicyclohexylamide, lithium hexamethyldisilazide, and the like; alkali metal alkoxides such as potassium tert-butoxide, sodium tert-butoxide, sodium methoxide, sodium ethoxide, potassium methoxide, potassium ethoxide, and the like. Sodium hydride is preferable. Sodium hydride may be added dropwise to the reaction system in the form of being dispersed in a mineral oil such as liquid paraffin.

The amount of the base used in Step (a) is usually from 0.7 mol to 1.3 mol, preferably from 0.8 mol to 1.2 mol, and more preferably from 0.9 mol to 1.1 mol, per 1 mol of a trimethyloxosulfonium salt or a trimethylsulfonium salt in view of the prevention of unnecessary residues of the trimethyloxosulfonium salt or the trimethylsulfonium salt and a base, in view of the prevention of side reaction, in view of the yield, in view of the quality, and the like.

Examples of the trimethyloxosulfonium salt include trimethyloxosulfonium chloride, trimethyloxosulfonium bromide, trimethyloxosulfonium iodide, trimethyloxosulfonium methylsulfate, and the like. Trimethyloxosulfonium bromide or trimethyloxosulfonium iodide is preferable in view of availability.

Examples of the trimethylsulfonium salt include trimethylsulfonium chloride, trimethylsulfonium bromide, trimethylsulfonium iodide, trimethylsulfonium methylsulfate, and the like. Trimethylsulfonium bromide or trimethylsulfonium iodide is preferable in view of availability.

The amount of the trimethyloxosulfonium salt or trimethylsulfonium salt used in Step (a) is usually from 0.8 to 2 mol, preferably from 1 to 1.6 mol, and more preferably from 1.1 to 1.5 mol, per 1 mol of the compound (I) in view of yield and effect corresponding to the amount.

The solvent used in Step (a) may be those which do not inhibit the reaction and examples thereof include ethers such as tetrahydrofuran (THF), methyl tert-butyl ether (MTBE), 1,4-dioxane, diethylene glycol dimethyl ether (diglyme), ethylene glycol dimethyl ether, 1,3-dioxolane, 2-methyltetrahydrofuran, and the like; aprotic polar solvents such as N,N-dimethylformamide (DMF), N,N-dimethylacetamide (DMAc), dimethylsulfoxide (DMSO), sulfolane, N-methyl-2-pyrolidinone (NMP), 1,3-dimethyl-2-imidazolidinone (DMI), N,N'-dimethylpropyleneurea (DMPU), hexamethylphosphoric amide (HMPA), nitrobenzene, carbon disulfide, acetonitrile, propionitrile, and the like; halogen-based solvents such as methylene chloride, 1,2-dichloroethane, monochlorobenzene, 1,2-dichlorobenzene, 2-chlorotoluene, 3-chlorotoluene, 4-chlorotoluene, 2-chloro-m-xylene, 2-chloro-p-xylene, 4-chloro-o-xylene, 2,3-dichlorotoluene, 2,4-dichlorotoluene, 2,5-dichlorotoluene, 2,6-dichlorotoluene, 3,4-dichlorotoluene, monofluorobenzene, and the like; aromatic hydrocarbons such as toluene, xylene, and the like; or solvent mixtures thereof. DMSO or a solvent mixture of DMSO and toluene, a solvent mixture of DMSO and THF, a solvent mixture of DMSO and MTBE, THF or MTBE is preferable. The amount of the solvent is usually from 1 to 50 L, preferably from 3 to 30 L, and more preferably from 5 to 15 L, per 1 kg of the compound (I).

The reaction temperature in Step (a) depends on the reagents to be used, and is usually from -30 to 80°C, preferably from 0 to 50°C, and more preferably from 10 to 40°C. The reaction time is usually from 0.5 to 48 hours, and preferably from 1 to 24 hours.

The compound (II) obtained in Step (a) can be isolated and purified by a conventional method. For example, the compound (II) can be isolated by pouring the reaction solution into water, followed by liquid separation, washing of the organic layer, drying and further concentration under reduced pressure. After the isolation, the compound (II) can also be purified, for example, by silica gel column chromatography. Alternately, the compound (II) can be subjected to the following reaction without purification.

The compound (II) obtained in Step (a) is a novel compound and is a useful intermediate of an antifungal agent or an epoxytriazole compound (VII).

### 2. Step (b)

The step (b) is the step for obtaining the compound (IV) by deprotecting the compound (II). For example, the compound (IV) can be obtained by reacting the compound (II) with an acid in a solvent.

The acid used in Step (b) is not limited, and examples thereof include inorganic acids such as hydrochloric acid, sulfuric acid, phosphoric acid, perchloric acid, and the like; and organic acids such as methanesulfonic acid, p-toluenesulfonic acid, benzenesulfonic acid, p-toluenesulphonic pyridinium salt (PPTS), and the like. Methanesulfonic acid or p-toluenesulfonic acid is preferable. The amount of the acid is usually from 0.0001 to 0.1 mol, preferably from 0.001 to 0.1 mol, and more preferably from 0.001 to 0.01 mol, per 1 mol of the compound (II) in view of the reaction rate and the prevention of side reaction such as ring-open of epoxide.

The solvent used in Step (b) may be those which do not inhibit the reaction. Examples thereof include water; aromatic hydrocarbons such as toluene, xylene, and the like; alcohols such as methanol, ethanol, 1-propanol, 2-propanol, ethylene glycol, and the like; ketones such as acetone, methyl ethyl ketone, methyl iso-butyl ketone, and the like; or solvent mixtures thereof. A solvent mixture of water, toluene and methanol, or methanol is preferable. The amount of the solvent is usually from 0.5 to 30 L, preferably from 1 to 15 L, and more preferably from 1 to 10 L, per 1 kg of the compound (II).

The reaction temperature in Step (b) depends on the reagent, and is usually from -20 to 100°C, preferably from 0 to 60 °C, and more preferably from 10 to 40°C. The reaction time is usually from one minute to 8 hours, and preferably from 3 minutes to 3 hours.

The compound (IV) obtained in Step (b) can be isolated and purified by a conventional method. For example, the compound (IV) can be isolated by pouring the reaction solution into water, followed by liquid separation, washing of the organic layer, drying and further concentration under reduced pressure. After the isolation, the compound (IV) can also be purified by silica gel column chromatography. Alternately, the compound (IV) can be subjected to the following reaction without purification.

### 3. Step (c)

Step (c) is the step for obtaining the compound (VI) by reacting the compound (IV) with the compound (V). For example, the compound (VI) can be obtained by reacting the compound (IV) with the compound (V) in a solvent in the presence of a base. The order of the addition of reagents is not specifically limited. For example, there may be employed a method comprising the steps of charging the compound (IV) and a base in a solvent and adding the compound (V) thereto, or a method of charging the compound (IV) in a solvent and the compound (V) and adding a base thereto.

Examples of the base used in Step (c) include aliphatic tertiary amines (e.g. trimethylamine, triethylamine, tributylamine, diisopropylethylamine, N-methylmorpholine, etc.), aromatic amines (e.g. pyridine, picoline, 2,6-lutidine, collidine, 4-(N,N-dimethylamino)pyridine, N,N-dimethylaniline, N,N-diethylaniline, etc.), alkali metal carbonates (e.g. sodium carbonate, potassium carbonate, etc.); basic ion exchange resins (e.g. Amber Light IRA-67, Amber Light IRA-900, etc.); and the like. Triethylamine and sodium carbonate are preferable and triethylamine is particularly preferable. The amount of the base is usually from 0.9 to 1.6 mol, preferably from 0.95 to 1.4 mol, and more preferably from 1 to 1.3 mol, per 1 mol of the compound (V) in view of the prevention of side reaction, in view of reaction rate, in view of the effect corresponding to the amount, and the like.

The amount of the compound (V) used in Step (c) is usually from 0.8 to 1.6 mol, preferably from 0.9 to 1.4 mol, and more preferably from 1 to 1.2 mol, per 1 mol of the compound (IV) in view of yield, efficiency, the prevention of side reaction, and the like.

The solvent used in Step (c) may be those which do not inhibit the reaction. Examples thereof include methylene chloride, 1,2-dichloroethane, monochlorobenzene, 1,2-dichlorobenzene, 2-chlorotoluene, 3-chlorotoluene, 4-chlorotoluene, 2-chloro-m-xylene, 2-chloro-p-xylene, 4-chloro-o-xylene, 2,3-dichlorotoluene, 2,4-dichlorotoluene, 2,5-dichlorotoluene, 2,6-dichlorotoluene, 3,4-dichlorotoluene, monofluorobenzene, nitrobenzene, carbon disulfide, toluene, acetonitrile, propionitrile, MTBE, ethylene glycol dimethyl ether, diglyme, THF, 2-methyltetrahydrofuran, 1,3-dioxolane, 1,4-dioxane, and the like, or solvent mixtures thereof. Toluene, acetonitrile, MTBE or THF is preferable. The amount of the solvent is usually from 1 to 50 L, preferably from 2 to 30 L, and more preferably from 3 to 15 L, per 1 kg of the compound (IV).

The reaction temperature in Step (c) depends on the reagents, and the like, and is usually from -20 to 80°C, preferably from 0 to 50°C, and more preferably from 10 to 40°C. The reaction time is usually from 0.5 to 24 hours, and preferably from 1 to 16 hours.

The compound (VI) obtained in Step (c) can be isolated and purified by a conventional method. For example, the compound (VI) can be isolated by pouring the reaction solution into water, followed by liquid separation, washing of the organic layer, drying and further concentration under reduced pressure. After the isolation, the compound (VI) can also be purified by silica gel column chromatography. Alternately, the compound (VI) can be subjected to the following reaction without purification.

### 4. Step (d)

Step (d) is the step for obtaining the epoxytriazole compound (VII) or a salt thereof by reacting the compound (VI) with 1,2,4-triazole. For example, the epoxytriazole compound (VII) can be obtained by reacting the compound (VI) with 1,2,4-triazole in a solvent in the presence of a base. The order of the addition of reagents is not specifically limited. For example, there may be employed a method comprising the steps of charging 1,2,4-triazole and a base in a solvent and adding the compound (VI) thereto, or a method comprising the steps of charging 1,2,4-triazole in a solvent, adding a base thereto to react them, and adding the reaction solution to a solution obtained by dissolving the compound (VI) in a solvent.

The base used in Step (d) is not limited as long as it forms a stable salt with 1,2,4-triazole. Examples thereof include alkali metal hydroxide such as sodium hydroxide, potassium hydroxide, lithium hydroxide, and the like; alkali metal carbonates such as potassium carbonate, sodium carbonate, lithium carbonate, cesium carbonate, and the like; alkali metal hydrides such as sodium hydride, potassium hydride, lithium hydride, and the like; alkyl alkali metals such as n-butyl lithium, methyl lithium, n-hexyl lithium, and the like; alkali metal amides such as sodium amide, potassium amide, lithium diisopropylamide, lithium dicyclohexylamide, lithium hexamethyldisilazide, and the like; alkali metal alkoxides such as potassium tert-butoxide, sodium tert-butoxide, potassium methoxide, sodium methoxide, sodium ethoxide, potassium ethoxide, and the like. Sodium hydride, potassium carbonate and sodium methoxide are preferable. Sodium hydride may be dispersed in an mineral oil such as liquide paraffin and the dispersion may be added dropwise in the reaction system. The amount of the base is usually from 0.7 mol to 2 mol, preferably from 0.8 mol to 1.5 mol, and more preferably from 0.85 mol to 1 mol, per 1 mol of 1,2,4-triazole in view of the prevention of unnecessary residues of 1,2,4-triazole, the prevention of excess residues of the base, the prevention of low yield, low quality due to side reaction, and the like.

The amount of 1,2,4-triazole used in Step (d) is usually from 0.9 to 2 mol, preferably from 1 to 1.6 mol, and more preferably 1.1 to 1.5 mol, per 1 mol of the compound (VI) in view of yield and the prevention of unnecessary residues of 1,2,4-triazole.

In Step (d), so as to promote the reaction, there may be added a so-called phase transfer catalyst, for example, tetraalkylammonium salts such as octadecyltrimethylammonium bromide, tetrabutylammonium sulfate, tetrabutylammonium bromide, tetrabutylammonium iodide, tetrabutylammonium chloride, and the like; and trialkylbenzylammonium salts such as benzyltrimethylammonium bromide, benzyltrimethylammonium chloride, benzyltriethylammonium chloride, and the like.

The solvent used in Step (d) may be those which do not inhibit the reaction. Examples thereof include ethers such as THF, MTBE, 1,4-dioxane, diglyme, ethylene glycol dimethyl ether, 1,3-dioxolane, 2-methyltetrahydrofuran, and the like; aprotic polar solvents such as DMF, DMAc, DMSO, sulfolane, NMP, DMI, HMPA, methyl isobutyl ketone, methyl ethyl ketone, acetone, cyclohexanone, 3-pentanone, nitrobenzene, carbon disulfide, acetonitrile, propionitrile, and the like; halogen-based solvents such as methylene chloride, 1,2-dichloroethane, monochlorobenzene, 1,2-dichlorobenzene, 2-chlorotoluene, 3-chlorotoluene, 4-chlorotoluene, 2-chloro-m-xylene, 2-chloro-p-xylene, 4-chloro-o-xylene, 2,3-dichlorotoluene, 2,4-dichlorotoluene, 2,5-dichlorotoluene, 2,6-dichlorotoluene, 3,4-dichlorotoluene, monofluorobenzene, and the like; aromatic hydrocarbons such as toluene, xylene, and the like; or solvent mixtures thereof. DMF, DMAc, DMI or THF is preferable.

The amount of the solvent is usually from 1 to 30 L, preferably from 2 to 20 L, and more preferably from 2 to 10 L, per 1 kg of the compound (VI).

The reaction temperature in Step (d) depends on the reagent, and is usually from 0 to 100°C, preferably from 20 to 80°C, and more preferably from 40 to 75°C. The reaction time is usually from 0.5 to 24 hours, and preferably from 1 to 12 hours.

The epoxytriazole compound (VII) obtained in Step (d) can be isolated and purified by a conventional method. For example, the epoxytriazole compound (VII) can be isolated by pouring the reaction solution into water, followed by liquid separation, washing of the organic layer, drying and further concentration under reduced pressure. After the isolation, the epoxytriazole compound (VII) can be purified by silica gel column chromatography or recrystallization. Alternately, the epoxytriazole compound (VII) can be subjected to the reaction which leads to the objective pharmaceutical product without purification.

### 5. Step (e)

The step (e) is the step for obtaining the compound (III) or a salt thereof by reacting the compound (II) with 1,2,4-triazole. For example, the compound (III) can be obtained by reacting the compound (II) with 1,2,4-triazole in a solvent in the presence of a base. The order of the addition of reagents is not specifically limited. For example, there may be employed a method comprising the steps of charging 1,2,4-triazole and a base in a solvent and adding the compound (II) thereto, or a method comprising the steps of charging 1,2,4-triazole in a solvent, adding a base thereto to react them and adding the reaction solution to a solution obtained by dissolving the compound (II) in a solvent.

The base used in Step (e) is not limited as long as it form a stable salt with 1,2,4-triazole. Examples thereof include alkali metal hydroxides such as sodium hydroxide, potassium hydroxide, lithium hydroxide, and the like; alkali metal carbonates such as potassium carbonate, sodium carbonate, lithium carbonate,cesium carbonate, and the like; alkali metal hydrides such as sodium hydride, potassium hydride, lithium hydride, and the like; alkyl alkali metals such as n-butyl lithium, methyl lithium, n-hexyl lithium, and the like; alkali metal amides such as sodium amide, potassium amide, lithium diisopropylamide, lithium dicyclohexylamide, lithium hexamethyldisilazide, and the like; and alkali metal alkoxides such as potassium tert-butoxide, sodium tert-butoxide, potassium methoxide, sodium methoxide, sodium ethoxide, potassium ethoxide, and the like. Sodium hydride, potassium carbonate and sodium methoxide are preferable. Sodium hydride may be added dropwise to the reaction system in the form of being dispersing in a mineral oil such as liquid paraffin. The amount of the base is usually from 0.4 to 1.5 mol, preferably from 0.5 mol to 1.1 mol, and more preferably from 0.6 mol to 1 mol, per 1 mol of 1,2,4-triazole in view of the prevention of unnecessary residues of 1,2,4-triazole or base, the prevention of low yield and low quality due to side reaction, and the like.

The amount of 1,2,4-triazole in Step (e) is usually from 1 to 10 mol, preferably from 1.5 to 7 mol, and preferably from 2 to 5 mol, per 1 mol of the compound (II) in view of yield, the prevention of unnecessary residues of 1,2,4-triazole, and the like.

In the step (e), so as to promote the reaction, there may be added a so-called phase transfer catalyst, for example, tetraalkylammonium salts such as octadecyltrimethylammonium bromide, tetrabutylammonium sulfate, tetrabutylammonium bromide, tetrabutylammonium iodide, tetrabutylammonium chloride, and the like; trialkylbenzylammonium salts such as benzyltrimethylammonium bromide, benzyltrimethylammonium chloride, benzyltriethylammonium chloride, and the like.

The solvent used in Step (e) may be those which do not inhibit the reaction. Examples thereof include ethers such as THF, MTBE, 1,4-dioxane, diglyme, ethylene glycol dimethyl ether, 1,3-dioxolane, 2-methyltetrahydrofuran, and the like; aprotic polar solvents such as DMF, DMAc, DMSO, sulfolane, NMP, DMI, HMPA, methyl isobutyl ketone, methyl ethyl ketone, acetone, cyclohexanone, 3-pentanone, nitrobenzene, carbon disulfide, acetonitrile, propionitrile, and the like; halogen-based solvents such as methylene chloride, 1,2-dichloroethane, monochlorobenzene, 1,2-dichlorobenzene, 2-chlorotoluene, 3-chlorotoluene, 4-chlorotoluene, 2-chloro-m-xylene, 2-chloro-p-xylene, 4-chloro-o-xylene, 2,3-dichlorotoluene, 2,4-dichlorotoluene, 2,5-dichlorotoluene, 2,6-dichlorotoluene, 3,4-dichlorotoluene, monofluorobenzene, and the like; aromatic hydrocarbons such as toluene, xylene, and the like; or solvent mixtures thereof. DMF, DMI, DMAc, THF or acetone is preferable. The amount of the solvent is usually from 1 to 30 L, preferably from 2 to 15 L, and more preferably from 2 to 10 L, per 1 kg of the compound (II).

The reaction temperature in Step (e) depends on the reagent, and is usually from 30 to 150°C, preferably from 50 to 120°C, and more preferably from 70 to 100°C. The reaction time is usually from 1 to 24 hours, preferably from 3 to 12 hours.

The compound (III) obtained in Step (e) can be isolated and purified by a conventional method. For example, the compound (III) can be isolated by pouring the reaction solution in water, followed by liquid separation, washing of the organic layer, drying and further concentration under reduced pressure. After the isolation, the compound (III) can also be purified by silica gel column chromatography, and the like. Alternately, the compound (III) can be subjected to the following reaction without purification.

The compound (III) obtained in Step (e) is a novel compound and is a useful intermediate for an antifungal agent or the epoxytriazole compound (VII).

### 6. Step (f)

Step (f) is the step for obtaining the compound (VIII) or a salt thereof by deprotecting the compound (III) or a salt thereof. For example, the compound (VIII) can be obtained by reacting the compound (III) with an acid in a solvent.

The acid used in Step (f) is not specifically limited. Examples thereof include inorganic acids such as hydrochloric acid, sulfuric acid, phosphoric acid, perchloric acid, and the like; and organic acids such as methanesulfonic acid, p-toluenesulfonic acid, benzenesulfonic acid, PPTS, and the like. Methanesulfonic acid, hydrochloric acid or p-toluenesulfonic acid is preferable. The amount of the acid is usually from 0.0001 to 0.1 mol, preferably from 0.001 to 0.1 mol, and more preferably from 0.001 to 0.01 mol, per 1 mol of the compound (III) in view of reaction rate, the prevention of side reaction, and the like.

The solvent used in Step (f) may be those which do not inhibit the reaction. Examples thereof include water; aromatic hydrocarbons such as toluene, xylene, and the like; alcohols such as methanol, ethanol, 1-propanol, 2-propanol, ethylene glycol, and the like; ketones such as acetone, methyl ethyl ketone, methyl isobutyl ketone, and the like; or solvent mixtures thereof. A solvent mixture of water, toluene and methanol, or methanol is preferable. The amount of the solvent is usually from 0.5 to 30 L, preferably from 1 to 15 L, and more preferably from 1 to 10 L, per 1 kg of the compound (III).

The reaction temperature in Step (f) depends on the reagent, and is usually from -20 to 100°C, preferably from 0 to 60°C, and more preferably from 10 to 40°C. The reaction time is usually from one minute to 8 hours, and preferably from 3 minutes to 3 hours.

The compound (VIII) obtained in Step (f) can be isolated and purified by a conventional method. For example, the compound (VIII) can be isolated by pouring the reaction solution in water, followed by liquid separation, washing of the organic layer, drying and further concentration under reduced pressure. After the isolation, the compound (VIII) can also be purified by silica gel column chromatography. Alternately, the compound (VIII) can be subjected to the following reaction without purification.

### 7. Step (g)

Step (g) is the step for obtaining the compound (IX) or a salt thereof by reacting the compound (VIII) or a salt thereof with the compound (V). For example, the compound (IX) can be produced by reacting the compound (VIII) with the compound (V) in a solvent in the presence of a base. The order of the addition of reagents is not specifically limited. For example, there may be employed a method comprising the steps of charging the compound (VIII) and a base in a solvent and adding the compound (V) thereto, or a method comprising the steps of charging the compound (VIII) and the compound (V) in a solvent and adding a base thereto.

Examples of the base used in Step (g) include aliphatic tertiary amines (e.g. trimethylamine, triethylamine, tributylamine, diisopropylethylamine, N-methylmorpholine, etc.), aromatic amines (e.g. pyridine, picholine, 2,6-lutidine, collidine, 4-(N,N-dimethylamino)pyridine, N,N-dimethylaniline, N,N-diethylaniline, etc.), alkali metal carbonates (e.g. sodium carbonate, potassium carbonate, etc.), and basic ion exchange resins (e.g. Amber Light IRA-67, Amber Light IRA-900, etc.), and the like. Triethylamine and sodium carbonate is preferable, and triethylamine is particularly preferable. The amount of the base is usually from 0.9 to 1.8 mol, preferably from 1.0 to 1.5 mol, and more preferably from 1.1 to 1.4 mol, per 1 mol of the compound (V) in view of the prevention of side reaction, the prevention of low reaction rate, the effect corresponding to the amount, and the like.

The amount of the compound (V) used in Step (g) is usually from 0.8 to 1.6 mol, preferably from 0.9 to 1.4 mol, and more preferably from 1 to 1.2 mol, per 1 mol of the compound (VIII) in view of yield, efficiency, the prevention of side reaction, and the like.

The solvent used in Step (g) may be those which do not inhibit the reaction. Examples thereof include methylene chloride, 1,2-dichloroethane, monochlorobenzene, 1,2-dichlorobenzene, 2-chlorotoluene, 3-chlorotoluene, 4-chlorotoluene, 2-chloro-m-xylene, 2-chloro-p-xylene, 4-chloro-o-xylene, 2,3-dichlorotoluene, 2,4-dichlorotoluene, 2,5-dichlorotoluene, 2,6-dichlorotoluene, 3,4-dichlorotoluene, monofluorobenzene, nitrobenzene, carbon disulfide, toluene, acetonitrile, propionitrile, MTBE, ethylene glycol dimethyl ether, diglyme, THF, 2-methyltetrahydrofuran, 1,3-dioxolane, 1,4-dioxane, and the like, or solvent mixtures thereof. A solvent mixture of toluene and THF, acetonitrile, MTBE or THF is preferable. The amount of the solvent is usually from 1 to 50 L, preferably from 2 to 30 L, and more preferably from 2 to 15 L, per 1 kg of the compound (VIII).

The reaction temperature in Step (g) depends on the reagents, and the like, and is usually from -20 to 80°C, preferably from 0 to 50°C, and more preferably from 10 to 40°C. The reaction time is usually from 10 minutes to 10 hours, and preferably from 10 minutes to 3 hours.

The compound (IX) obtained in Step (g) can be isolated and purified by a conventional method. For example, the compound (IX) can be isolated by pouring the reaction solution into water, followed by liquid separation, washing of the organic layer, drying and further concentration under reduced pressure. After the isolation, the compound (IX) can also be purified by silica gel column chromatography. Alternately, the compound (IX) can be subjected to the following reaction without purification. Furthermore, a reaction mixture containing the compound (IX) can also be provided for the next reaction without isolation and purification of the compound (IX).

### 8. Step (h)

Step (h) is the step for obtaining the epoxytriazole compound (VII) or a salt thereof by treating the compound (IX) or a salt thereof with a base. The step can be conducted by reacting the compound (IX) with a base in a solvent. The step can also be conducted by adding a base to the reaction mixture containing the compound (IX) in Step (g). In such case, the amount of the samples used in Step (h) may be within the following range based on the compound (VIII) instead of the compound (IX).

The base used in Step (h) is not specifically limited. Examples thereof include sodium hydroxide, potassium hydroxide, sodium methoxide, sodium ethoxide, sodium tert-butoxide, potassium methoxide, potassium ethoxide, potassium tert-butoxide, sodium carbonate, potassium carbonate, and the like. Sodium hydroxide or sodium methoxide is preferable. The amount of the base is usually from 0.9 to 5 mol, preferably from 1 to 4 mol, and more preferably from 1.2 to 3.5 mol, per 1 mol of the compound (IX) in view of the completion of the reaction, the effect corresponding to the amount, and the like.

The solvent used in Step (h) may be those which do not inhibit the reaction, and examples thereof include water, methylene chloride, 1,2-dichloroethane, monochlorobenzene, 1,2-dichlorobenzene, 2-chlorotoluene, 3-chlorotoluene, 4-chlorotoluene, 2-chloro-m-xylene, 2-chloro-p-xylene, 4-chloro-o-xylene, 2,3-dichlorotoluene, 2,4-dichlorotoluene, 2,5-dichlorotoluene, 2,6-dichlorotoluene, 3,4-dichlorotoluene, monofluorobenzene, nitrobenzene, carbon disulfide, toluene, acetonitrile, propionitrile, MTBE, ethylene glycol dimethyl ether, diglyme, THF, 2-methyltetrahydrofuran, 1,3-dioxolane, 1,4-dioxane, and the like, or solvent mixtures thereof. A solvent mixture of toluene and THF, toluene, THF or MTBE is preferable. The amount of the solvent is usually from 1 to 30 L, preferably from 2 to 20 L, and more preferably from 2.5 to 10 L, per 1 kg of the compound (IX).

The reaction temperature in Step (h) depends on the reagent, and is usually from -30 to 80°C, preferably from -10 to 50°C, and more preferably from 0 to 30°C. The reaction time is usually from 0.5 to 12 hours, and preferably from 0.5 to 6 hours.

The epoxytriazole compound (VII) obtained in Step (h) can be isolated and purified by a conventional method. For example, the epoxytriazole compound (VII) can be isolated by pouring the reaction solution into water, followed by liquid separation, washing of the organic layer, drying and further concentration under reduced pressure. After the isolation, the epoxytriazole compound (VII) can also be purified by silica gelcolumn chromatography and recrystallization. Alternately, the epoxytriazole compound (VII) can be subjected to the reaction leading to the objective pharmaceutical product without purification. For example, the epoxytriazole compound (VII) can be converted into a triazole compound useful as an antifungal agent according to the methods described in Patent Document 1 and Patent Document 3.

The compound (I) as a starting material of the present invention is a novel compound and can be produced by the method including the following steps (i), (j), (k) and (1) as shown in the following reaction scheme:
Step (i): the step for obtaining the aryl magnesium halide (XII) by reacting Grignard reagent (X) with the compound (XI);
Step (j): the step for obtaining the compound (XIV) by reacting the obtained arylmagnesium halide (XII) with the compound (XIII);
Step (k): the step for obtaining the compound (XV) by deprotecting the obtained compound (XTV); and
Step (1): the step for obtain the compound (I) by reacting the resulting compound (XV) with 2-methoxypropene; 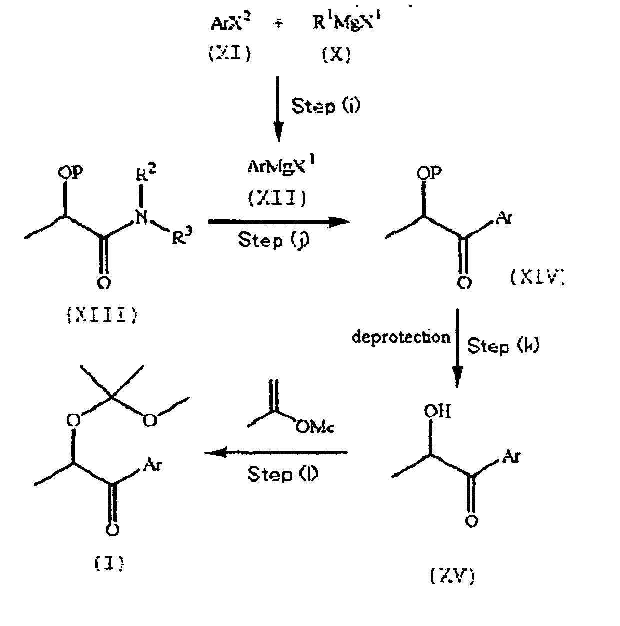
wherein P represents a protective group for a hydroxyl group; R² and R³ each independently represents an alkyl group having 1 to 12 carbon atoms or an alkoxy group having 1 to 8 carbon atoms, or R² and R³ bond to form divalent acyclic hydrocarbon group which forms aliphatic heterocycle together with adjacent nitrogen atom, and one -CH₂- in the divalent acyclic hydrocarbon group may be substituted by -O-, with the proviso that R² and R³ don't represent alkoxy groups having 1 to 8 carbon atoms simultaneously; Ar represents difluorophenyl group; R¹ represents hydrocarbon group; and X¹ and X² each independently represents halogen atom.

Examples of the halogen atom represented by X¹ and X² include chlorine, bromine, iodine, and the like. Chlorine and bromine are preferable.

Examples of the hydrocarbon group represented by R¹ include aryl groups, linear or branched alkyl groups having 1 to 8 carbon atoms (e.g. methyl, ethyl, propyl, isopropyl, butyl, isobutyl, sec-butyl, tert-butyl, pentyl, isopentyl, sec-pentyl, tert-pentyl, hexyl, heptyl, octyl, etc.), cycloalkyl groups (e.g. cyclopropyl, cyclobutyl, cyclopentyl, cyclohexyl, etc.), linear or branched alkenyl groups having 2 to 6 carbon atoms (e.g. vinyl, allyl, 1-propenyl, etc.), and the like. Isopropyl, isobutyl or tert-butyl is preferable.

The alkyl group as for R² and R³ may be linear or branched alkyl groups having 1 to 12 carbon atoms, and preferably 1 to 3 carbon atoms, and examples thereof include methyl, ethyl, propyl, isopropyl, butyl, isobutyl, sec-butyl, tert-butyl, pentyl, isopentyl, neopentyl, hexyl, heptyl, octyl, nonyl, decyl, undecyl, dodecyl, and the like. Methyl is preferable.

The alkoxy groups as for R² and R³ may be linear or branched alkoxy groups having 1 to 8 carbon atoms, and preferably 1 to 3 carbon atoms, and examples thereof include methoxy, ethoxy, propoxy, isopropoxy, butoxy, isobutoxy, sec-butoxy, tert-butoxy, pentyloxy, isopentyloxy, neopentyloxy, hexyloxy, heptyloxy, octyloxy, nonyloxy, decyloxy, undecyloxy, dodecyloxy, and the like. Methoxy is preferable.

In that case, R² and R³ are not simultaneously alkoxy groups. It is preferred that one of them is an alkyl group and other one is alkoxy group so as to prevent the aryl magnesium halide (XII) from further reacting with the compound (XIV).

When R² and R³ are combined and represent divalent acyclic hydrocarbon group which forms aliphatic heterocycle together with the adjacent nitrogen atom, example of such divalent aliphatic heterocycle include tetramethylene group, pentamethylene group, hexamethylene group, and the like. One of -CH₂- in the divalent acyclic hydrocarbon group may be substituted with -O-. Examples of the group in which one of -CH₂- in the divalent acyclic hydrocarbon group is substituted with -O- include -O-CH₂-CH₂-CH₂-group, -CH₂-O-CH₂-CH₂- group, -O-CH₂-CH₂-CH₂-CH₂- group, -CH₂-O-CH₂-CH₂-CH₂- group, -CH₂-CH₂-O-CH₂-CH₂- group, -O-CH₂-CH₂-CH₂-CH₂-CH₂- group, -CH₂-O-CH₂-CH₂-CH₂-CH₂- group, -CH₂-CH₂-O-CH₂-CH₂-CH₂- group, and the like. When R² and R³ are combined and represent divalent aliphatic hydrocarbon group, in which one of -CH₂- may be substituted with -O-, examples of the aliphatic heterocycle formed together with the adjacent nitrogen atom include pyrrolidine ring, piperidine ring, homopiperidine ring, morpholine ring, and the like. Morpholine ring is preferable.

The protective group represented by P for hydroxyl group is not limited as long as it does not react with the Grignard reagent (X). Examples thereof include acetal-based protective groups such as methoxymethyl, benzyloxymethyl, ethoxymethyl, (1-methoxy-1-methyl)ethyl, 1-ethoxyethyl, 1-butoxyethyl, 1-benzyloxyethyl, 2-tetrahydropyranyl, 2-tetrahydrofuranyl, 4-methoxytetrahydropyranyl, and the like; silyl ether-based protective groups such as trimethylsilyl, triethylsilyl, toriisopropylsilyl, tri-n-butylsilyl, tertbutyldimethylsilyl, tert-butyldiphenylsilyl, and the like. Acetal-based protective group is preferable, and 2-tetrahydropyranyl, (1-methoxy-1-methyl)ethyl, 1-ethoxyethyl and 2-tetrahydrofuranyl are more preferable.

When the compounds (XIII), (XIV) and (XV) contain one or more asymmetric carbon atoms, these compounds may be any optically active isomers or a mixture thereof (for example, racemic mixture, enantiomer mixture, and the like). Compounds having preferable configuration are compounds represented by the following general formulas (XIIIa), (XIVa) and (XVa) or enantiomers thereof: 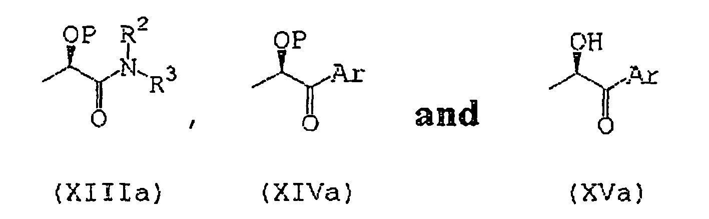 wherein P, R², R³ and Ar have the same meanings as defined above.

Furthermore, when the protective group represented by P for hydroxyl group is (1-methoxy-1-methyl)ethyl group, the compound (I) which is equal to the compound (XIV) in which P is (1-methoxy-1-methyl)ethyl group can be obtained by only Step (i) and Step (j) without requiring Step (k) and Step (1).

The process for producing the compound (XIV) is described in JP H04-356471-A, JP H05-230038-A and "Chemical & Pharmaceutical Bulletin", The Pharmaceutical Society of Japan, 1993, Vol. 41, No. 6, p.1035-1042. In the processes described in these document, the Grignard reagent corresponding to the aryl magnesium halide (XII) i.e., 2,4-difluorophenyl magnesium bromide is prepared from 1-bromo-2,4-difluorobenzene and metal magnesium. However, there is a problem that the aryl magnesium halide (XII) is relatively unstable and therefore the decomposition may occur under usual reaction conditions for producing the Grignard reagent (reaction temperature: 40 to 50°C), resulting in low yield and coloration.

To the contrary, in Step (i), there is an advantage that the Grignard exchange reaction between the Grignard reagent (X) and the compound (XI) can be conducted under relatively moderate condition (reaction temperature: around -20 to 30°C), the decomposition of aryl magnesium halide (XII) can be reduced as possible.

Also there is a problem that the compound (XV) is chemically unstable and therefore purification by distillation promotes racemization and isomerization, resulting in the production of a compound as by-product represented by the general formula (XV') (hereinafter, also referred to as the compound (XV')): 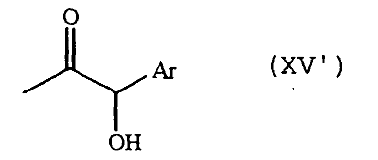 wherein Ar has the same meaning as defined above,
it is also made clear that the compound (XV) can be purified by recrystallization after converting into the compound (I).

Steps (i) to (1) will be now described.

### 9. Step (i)

The step (i) is the step for preparing the aryl magnesium halide (XII) by reacting the Grignard reagent (X) with the compound (XI) in a solvent. The order of the addition of reagents is not specifically limited. For example, there may be employed a method comprising the steps of adding the compound (XI) (preferably in the form or a solution) to the Grignard reagent (X) solution or vice versa.

The Grignard reagent (X) can be quantitatively prepared by a conventionally known method of preparing Grignard reagent, that is, it is prepared from a halogenated hydrocarbon (hereinafter, also referred to as the halogenated hydrocarbon (Xa)) represented by the general formula (Xa):

R¹-X¹

wherein R¹ and X¹ have the same meanings as defined above,
and metal magnesium in a solvent (e.g., THF, diethyl ether, MTBE, ethylene glycol dimethyl ether, etc.).

The amount of the Grignard reagent (X) (the converted amount from the amount of the above halogenated hydrocarbon (Xa) or magnesium) is usually from 0.7 mol to 1.5 mol, preferably from 0.8 mol to 1.3 mol, and more preferably from 0.9 mol to 1.2 mol, based on the compound (XI) in view of yield of the arylmagnesium halide (XII), the prevention of side reaction associated with excess residues of excess Grignard reagent, and the like.

The solvent used in Step (i) may be those which do not inhibit the reaction, and examples thereof include ethers such as THF, diethyl ether, MTBE, 1,4-dioxane, diglyme, ethylene glycol dimethyl ether, 1,3-dioxolane, 2-methyltetrahydrofuran, and the like; aromatic hydrocarbons such as toluene, xylene, and the like; or solvent mixtures thereof, and preferably THF, MTBE, ethylene glycol dimethyl ether. The amount of the solvent (including the amount of a solvent on preparing hydrocarbon halide (Xa)) is usually from 1 to 50 L, preferably from 3 to 30 L, and more preferably from 5 to 20 L, per 1 kg of the compound (XIII).

The reaction temperature in Step (i) is usually from -30 to 50°C, preferably from -20 to 30°C, and more preferably from -10 to 20°C. The reaction time is usually from 10 minutes to 3 hours, and preferably from 20 minutes to 2 hours.

The aryl magnesium halide (XII) prepared in Step (i) can be usually used for the following Step (j) as a reaction mixture.

### 10. Step (j)

Step (j) is a method for obtaining the compound (XIV) by reacting the aryl magnesium halide (XII) with the compound (XIII). For example, this step can be conducted by adding (preferably dropwise) a solution of the compound (XIII) into the reaction mixture in Step (i). The order of the addition may be reversed.

The compound (XIII) used in Step (j) can be synthesized by reacting a lactic ester derivative with amine represented by the general formula:

HNR²R³

wherein R² and R³ have the same meanings as defined above,
to obtain lactatic amide compound represented by the general formula: CH₃CH(OH)CONR²R³
wherein R² and R³ have the same meanings as defined above,
and protecting its hydroxyl group by a known method (for example, a method described in Protective Groups in Organic Synthesis Third Edition, p.27-49, Wiley-Interscience, John Wiley & Sons, Inc.).

The amount of the compound (XIII) in Step (j) is usually from 0.5 to 1.2 mol, preferably from 0.6 to 1 mol, and more preferably from 0.7 to 0.9 mol, per 1 mol of the compound (XI) used in Step (i) in view of the prevention of further addition of excess aryl magnesium halide (XII) to the obtained compound (XIV) and the prevention of residues of the compound (XIII).

The solvent used for the compound (XIII) solution in Step (j) is preferably the same as that used in Step (i). The amount of the solvent is usually from 1 to 50 L, preferably from 3 to 30 L, and more preferably from 5 to 20 L, per 1 kg of the compound (XIII).

The reaction temperature in Step (j) is usually from -20 to 70°C, preferably from 0 to 50°C, and more preferably from 10 to 40°C. The reaction time is usually from 30 minutes to 40 hours, and preferably from 1 to 20 hours.

The compound (XIV) obtained in Step (j) can be isolated and purified by a conventional method.

For example, the compound (XIV) can be isolated by pouring a reaction solution into acidic water (for example, aqueous ammonium chloride solution), followed by liquid separation, washing of the organic layer, drying and concentration under reduced pressure. The compound (XTV) can also be purified by silica gel column chromatography. Alternately, the compound (XIV) can be subjected to the following reaction without purification.

### 11. Step (k)

The step (k) is the step for obtaining the compound (XV) by deprotecting P of the compound (XIV). This step can be conducted by a conventionally known method for deprotecting P (for example, a method described in Protective Groups in Organic Synthesis Third Edition, p.27-49, Wiley-Interscience, John Wiley & Sons, Inc.). Deprotection of cetal protective group as a preferred aspect will now be described, but is not limited thereto.

As P is an acetal protective group, the deprotection can be conducted by treating the compound (XIV) with an acid in a solvent.

Examples of the acid include inorganic acids such as hydrochloric acid, sulfuric acid, phosphoric acid, perchloric acid, and the like; and organic acids such as methanesulfonic acid, p-toluene sulfonic acid, benzenesulfonic acid, PPTS, and the like. Methanesulfonic acid or p-toluenesulfonic acid is preferable. The amount of the acid is usually from 0.0001 to 0.1 mol, preferably from 0.001 to 0.1 mol, and more preferably from 0.005 to 0.05 mol, per 1 mol of the compound (XIV) in view of reaction rate, the prevention of racemization and isomerization of the compound (XV), and the like.

The solvent used in Step (k) may be those which do not inhibit the reaction, and examples thereof include water; aromatic hydrocarbons such as toluene, xylene, and the like; alcohols such as methanol, ethanol, ethylene glycol, 1-propanol, 2-propanol, and the like; ketones such as acetone, ethyl ethyl ketone, methyl isobutyl ketone, and the like; THF, and the like; or solvent mixtures thereof. A solvent mixture of water, toluene and ethylene glycol, methanol or ethanol is preferable. The amount of the solvent is usually from 1 L to 50 L, preferably from 3 L to 30 L, and more preferably from 5 L to 20 L, per 1 kg of the compound (XIV).

The reaction temperature in Step (k) depends on the reagent, and is usually from 0 to 80°C, preferably from 20 to 60°C, more preferably from 30 to 50°C. The reaction time is usually from 0.5 to 12 hours, preferably from 0.5 to 6 hours.

The compound (XV) obtained in Step (k) can be isolated and purified by a conventional method. For example, the compound (XV) can be isolated by pouring a reaction solution into water, followed by liquid separation, washing of the organic layer, drying and concentration under reduced pressure. After the isolation, the compound (XV) can be also purified by silica gel column chromatography and distillation under reduced pressure. Alternately, the compound (XV) can be subjected to the following reaction without purification.

### 12. Step (I)

Step (I) is the step for obtaining the compound (I) by reacting the compound (XV) with 2-methoxypropene. For example, the compound (I) can be obtained by reacting the compound (XV) with 2-methoxypropene in a solvent in the presence of an acid. The order of the addition of reagents is not specifically limited, and the respective materials may be added to the reaction system sequentially or simultaneously.

The amount of 2-methoxypropene used in Step (1) is usually from 1 mol to 2 mol, preferably from 1.1 mol to 1.6 mol, and more preferably from 1.2 mol to 1.5 mol, per 1 mol of the compound (XV) in view of reaction rate, the prevention of side reaction, and the like.

The acid used in the step (1) is not specifically limited and examples thereof include inorganic acids such as hydrochloric acid, sulfuric acid, phosphoric acid, perchloric acid, and the like; and organic acids such as methanesulfonic acid, p-toluenesulfonic acid, benzenesulfonic acid, PPTS, and the like. PPTS, p-toluenesulfonic acid or methanesulfonic acid is preferable. The amount of the acid is usually from 0.0001 to 0.1 mol, preferably from 0.001 to 0.1 mol, and more preferably from 0.005 to 0.05 mol, per 1 mol of the compound (XV) in view of reaction rate, the prevention of side reaction, and the like.

The solvent used in Step (1) may be those which do not inhibit the reaction. Examples thereof include ethers such as THF, MTBE, 1,4-dioxane, diglyme, ethylene glycol dimethyl ether, and the like; hydrocarbons such as toluene, xylene, hexane, and the like; or solvent mixtures thereof. MTBE, toluene or THF is preferable. The amount of the solvent is usually from 0.5 to 30 L, preferably from 1 to 20 L, and more preferably from 2 to 15 L, per 1 kg of the compound (XV).

The reaction temperature in Step (1) depends on the reagent, and is usually from -20 to 80°C, preferably from -10 to 50°C, and more preferably from 0 to 40°C. The reaction time is usually from 0.5 to 12 hours, and preferably from 1 to 6 hours.

The compound (I) obtained in Step (1) can be isolated and purified by a conventional method. For example, the compound (I) can be isolated by pouring the reaction solution into water, followed by liquid separation, washing of the organic layer, drying and concentration under reduced pressure. After the isolation, the compound (I) may be isolated by silica gel column chromatography, but it is preferable to be purified by recrystallization from crystallization solvents (for example, ethers such as diethyl ether, THF, etc.; acetone; acetonitrile; hydrocarbon-based solvent such as toluene, benzene, hexane, heptane, etc.; alcohols such as methanol, ethanol, isopropanol, etc.; or solvent mixtures thereof).

The present invention will now be described in detail by way of examples. The present invention is not limited to thereto.

### Reference Example 1: (2R)-2',5'-difluoro-2-hydroxypropiophenone

### (a): (D)-N,N-dimethyllactamide

N,N-dimethylamine (405.7 g, 9.0 mol) was blown into a solution of (D)- methyl lactate (469 g, 4.5 mol) in methanol (234 mL) at 0 to 15°C and the mixture was stirred for 24 hours in an airtight container at 60 to 65°C and concentrated under reduced pressure to obtain 525 g of (R)-N,N-dimethyllactamide.

### (b): (2R)-N,N-dimethyl-2-O-(1-ethoxyethyl)lactamide

Methanesulfonic acid (0.19 g, 2 mmol) and ethyl vinyl ether (15.9 g, 0.22 mol) were sequentially added dropwise to a solution of a portion of the (D)-N,N-dimethyllactamide obtained in (a) (23.4 g, 0.2 mol) in THF (23.4 mL) at 15 to 20°C and the mixture was stirred for 5 hours to obtain a solution of (2R)-N,N-dimethyl-2-O-(1-ethoxyethyl)lactamide in THF.

### (c) Preparation of 2,5-difluorophenylmagnesium bromide

Iodine (small amount) was added to a mixed liquid of magnesium (6.32 g, 0.26 mol) and THF (19 ml) and then 2-bromopropane (35.2 g, 0.286 mol) in THF (94 ml) was added dropwise thereto at 20 to 30°C over about 2.5 hours. After stirring for 30 minutes, the resulting slurry of isopropyl magnesium bromide was slowly added dropwise to a solution of 2,5-difluorobromobenzene (50.2 g, 0.26 mol) in THF (50ml) at 0 to 5°C. Thereafter, the reaction mixture was stirred at room temperature for about 40 minutes and the reaction end was confirmed by HPLC analysis to obtain a solution of 2,5-difluorophenylmagnesium bromide in THF.

### (d): (2R)-2',5'-difluoro-2-(1'-ethoxyethoxy)propiophenone

The solution of (2R)-N,N-dimethyl-2-O-(1-ethoxyethyl)lactamide in THF (23.4 ml) obtained in (b) was added dropwise at room temperature to the solution of 2,5-difluorophenylmagnesium bromide in THF, which has been prepared in (c) and the mixture was stirred at room temperature overnight. The reaction solution was poured into a cooled aqueous ammonium chloride solution and the mixture was neutralized with an aqueous citric acid solution and then extracted with toluene three times. The combined organic layers were sequentially washed with an aqueous ammonium chloride solution and water to obtain a solution of (2R)-2',5'-difluoro-2-(1'-ethoxyethoxy)propiophenone in toluene.

### (e): (2R)-2',5'-difluoro-2-hydroxypropiophenone

Ethylene glycol (10.5 ml) and methanesulfonic acid (0.19 g, 2 mmol) were added to the solution of (2R)-2',5'-difluoro-2-(1'-ethoxyethoxy)propiophenone in toluene obtained in (d) and then the mixture was heated for 1 hour at 40 to 45°C. Since the reaction was not completed, methanesulfonic acid (0.19 g, 2 mmol) was further added thereby to complete the reaction. The reaction mixture was sequentially washed with 5% brine (one time) and water (two times) and then the organic layers were concentrated and the concentrate was distilled under reduced pressure to obtain (2R)-2',5'-difluoro-2-hydroxypropiophenone. Yield: 28.3 g (89-95°C/2 to 5 mmHg), 76.1% (based on (D)-N,N-dimethyllactamide).

### Reference Example 2: (2R)-2',4'-difluoro-2-hydroxypropiophenone (crude product)

The crude product of (2R)-2',4'-difluoro-2-hydroxypropiophenone was obtained by the same procedure as in Reference Example 1 except for using 2,4-difluorobromobenzene instead of 2,5-difluorobromobenzene in Reference Example 1(c) and conducting no distillation under reduced pressure in Reference example 1(e).

### Reference Example 3: (2R)-2',4'-difluoro-2-hydroxypropiophenone (product purified by distillation)

The product purified by distillation of (2R)-2',4'-difluoro-2-hydroxypropiophenone was obtained by the same procedure as in Reference example 1 except for using 2,4-difluorobromobenzene instead of 2,5-difluorobromobenzene in Reference Example 1(c).

### Example 1: (2R)-2',4'-difluoro-2-(1'-methoxy-1'-methylethoxy)propiophenone

A crude product of (2R)-2',4'-difluoro-2-hydroxypropiophenone obtained by Reference Example 2 (55.6 g, prepared from 0.3 mol of (D)-N,N-dimethyllactamide) was mixed with MTBE (110 ml) and the mixture was cooled to 0 to 10°C. Thereafter, pyridinium p-toluenesulfonate (PPTS) (0.75 g, 0.01 eq.) was added and subcsequently 2-methoxypropene (13.7 g, 1.1 eq.) was slowly added dropwise over about 1 hour. Then, the temperature was elevated to 10 to 20°C, PPTS (1.87 g, 0.025 eq.) and 2-methoxypropene (4.31 g, 0.2 eq.) were added and then the mixture was stirred for 2 hours. After the completion of the reaction, the reaction mass was sequentially washed with 5% sodium bicarbonate water (50ml) and water (50 ml x 2) and concentrated. The resulting concentrate (72.6 g) was purified by crystallization from a solvent mixture of toluene (14 ml) and heptane (28 ml) to obtain the title compound as a white solid. Yield: 44.0 g, 57.1% (based on (D)-N,N-dimethyllactamide). Melting point: 64.8-65.0 °C.
¹H-NMR(400MHz, in CDCl₃)d:1.19,1.42(each 3H, s), 1.40(3H, d, J=7Hz), 3.20(3H, s), 5.07(1H, q, J=7Hz), 6.89, 6.98, 7.94(each 1H, m).

### Example 2: (2R)-2',4'-difluoro-2-(1'-methoxy-1'-methylethoxy)propiophenone

The product purified by distillation of (2R)-2',4'-difluoro-2-hydroxypropiophenone obtained by the Reference Example 3 (4.02 g, 21.6 mmol) was mixed MTBE (20 ml) and the mixture was cooled to 0 to 10 °C. Thereafter, PPTS (0.27 g, 1.08 mmol, 0.05 eq.) and 2-methoxypropene (2.34 g, 32.4 mmol, 1.5 eq.) were sequentially added. After the completion of the reaction, the reaction mass was washed sequentially 5% sodium bicarbonate water (10 ml x 2), dried over anhydrous magnesium sulfate, and concentrated to obtain the title compound as a white solid. Yield: 5.50 g, 98.7%.

### Example 3: (2R)-2',4'-difluoro-2-(1'-methoxy-1'-methylethoxy)propiophenone (a): Isomerization of (2R)-2',4'-difluoro-2-hydroxypropiophenone

PPTS (55 mg, 0.22 mmol, 0.001 eq.) was added to the product purified by distillation of (2R)-2',4'-difluoro-2-hydroxypropiophenone obtained by the Reference Example 3 (41.0 g, 0.22 mol) and the mixture was isomerized by heating for 2 hours at 145°C of bath temperature to obtain a mixture of (2R)-2',4'-difluoro-2-hydroxypropiophenone containing 6% of 1-(2',4'-difluorophenyl)-1-hydroxyacetone.

### (b) (2R)-2',4'-difluoro-2-(1'-methoxy-1'-methylethoxy)propiophenone

The mixture obtained in (a) was cooled to 10 to 20°C. Then toluene (100 ml) and PPTS (1.10 g, 4.4 mmol, 0.02 eq.) were sequentially added thereto and then 2-methoxypropene (19.0 g, 0.26 mol,1.2 eq.) was slowly added dropwise over about 1.5 hours. After the completion of the reaction, triethylamine (1.53 ml,11 mmol) was added to the reaction mass and then the mixture was washed with water (50 ml x 2), dried over anhydrous magnesium sulfate, filtered and concentrated. The resulting concentrate (57.1 g) was purified by crystallization from a solvent mixture of toluene (15 ml) and heptane (15 ml) to obtain the title compound as a white solid. Yield: 34.0 g, 60.0%.

The resulting (2R)-2',4'-difluoro-2-(1'-methoxy-1'-methylethoxy)propiophenone was subjected to a reaction and work-up under the same conditions as in Reference Example 1(e) to obtain (2R)-2',4'-difluoro-2-hydroxypropiophenone.

### Example 4: (2R,3R)-3-(2',4'-difluorophenyl)-3,4-epoxy-2-(1'-methoxy-1'-methylethoxy)butane

Trimethylsulfoxonium iodide (33.2 g, 0.151 mol, 1.3 eq.) was dissolved in a solvent mixture of DMSO (180 ml) and THF (75 ml) and the solution was cooled to 10 to 15°C. Then, sodium hydride (60% dispersion in liquid paraffin, 4.87 g, 0.122 mol, 1.05 eq.) was added portionwise and the mixture was stirred until hydrogen generation stopped. Next, a solution of (2R)-2',4'-difluoro-2-(1'-methoxy-1'-methylethoxy)propiophenone (30.0 g, 0.116 mol) in a solvent mixture of DMSO (50 ml) and THF (30 ml) was added dropwise and the mixture was stirred at room temperature overnight. Since the material was remained, the reaction mass was cooled to 0 to 5°C and sodium hydride (60% dispersion in liquid paraffin, 1.16 g, 0.029 mol, 0.25 eq.) was added to complete the reaction. The reaction mixture was added dropwise to a mixed solution of water (255 ml) and toluene (120 ml). The mixture was extracted twice with toluene (120ml and 60 ml). The combined extracts were washed with water (120 ml) twice, and then concentrated under reduced pressure to obtain 34.0 g of a light yellow oil. The ratio of (2R,3R) form to (2R,3S) form was 4:1 (area percentage in HPLC).

### HPLC conditions

Column: Symmetry C18, 3.9 mm x 150 mm, 5 µm; column temperature: 35°C; mobile phase: 50% (v/v) CH₃CN-H₂O; flow rate: 1 ml/min; detection: UV 254 nm.
Retention time: (2R,3R) form: 9.4 min, (2R,3S) form: 9.9 min.
¹H-NMR(400MHz,in CDCl₃)d:
   (2R,3R) form :1.18(3H, dd, J=6Hz,1Hz), 1.36,1.40(each 3H, s), 2.81, 3.21(each 1H, d, J=6Hz), 3.26(3H, s), 4.09(1H, q, J=6Hz), 6.76-6.79(1H, m), 6.81-6.90(1H, m), 7.44(1H, m).
   (2R,3S) form:1.15(dd, J=6Hz,1Hz), 1.30, 1.32(each, s), 2.84, 3.13(each d, J=6Hz), 3.23(s), 4.00(q, J=6Hz), 6.76-6.79(m), 6.81-6.90(m), 7.44(m).

### Example 5: (2R,3S)-2-(2,4-difluorophenyl)-3-methyl-2-[(1H-1,2,4-triazol-1-yl)methyl]oxirane

### (a): (2R,3R)-3-(2',4'-difluorophenyl)-2-hydroxy-3,4-epoxybutane

The light yellow oil of (2R,3R)-3-(2',4'-difluorophenyl)-3,4-epoxy-2-(1'-methoxy-1'-methylethoxy)butane obtained in Example 4 (a mixture of (2R,3R) form and (2R,3S) form in 4:1, 34.0 g, 0.116 mol) was mixed with a mixed solution of toluene (60 ml), methanol (10 ml) and water (5 ml). Methanesulfonic acid (56 mg, 0.58 mmol, 0.005 eq.) was added thereto at room temperature and the mixture was stirred for 5 minutes. After the completion of the reaction, 5% sodium bicarbonate water (10 ml) and water (50 ml) were poured and the mixture was stirred and the layers were separated. The organic layer was washed with water (50 ml x 2) and concentrated to obtain 24.5 g of a mixture of (2R,3R)-3-(2',4'-difluorophenyl)-2-hydroxy-3,4-epoxybutane and its stereoisomer (2R, 3S) form (4:1) as an oil.

### (b): (2R,3R)-3-(2',4'-difluorophenyl)-3,4-epoxy-2-methanesulfonyloxybutane

The mixture of (2R,3R)-3-(2',4'-difluorophenyl)-2-hydroxy-3,4-epoxybutane and its stereoisomer (2R,3S) form (4:1) (24.5 g, 0.116 mol) obtained in (a) and triethylamine (13.5 g, 0.133 mol) were added to toluene (108 ml). After cooling (0 to 15 °C), methanesulfonyl chloride (14.6 g, 0.128 mol) was added dropwise and the mixture was stirred. After confirming the completion of the reaction by reverse-phase high performance liquid chromatography, the reaction mass was sequentially washed with water (60 ml x 2) and brine (60 ml). The resulting organic layer was dried with anhydrous magnesium sulfate, filtered and concentrated under reduced pressure to obtain about 32.3 g (0.116 mol) of an oily mixture of (2R,3R)-3-(2',4'-difluorophenyl)-3,4-epoxy-2-methanesulfonyloxybutane and its stereoisomer (2R, 3S) form (4:1).

### (c): (2R,3S)-2-(2,4-difluorophenyl)-3-methyl-2-[(1H-1,2,4-triazol-1-yl)methyl]oxirane

The total amount of the mixture obtained in (b) was dissolved in DMF (35 ml). A solution of sodium salt of 1,2,4-triazole prepared previously from 1,2,4-triazole (10.4 g, 0.151 mol) and sodium hydride (60% dispersion in liquid paraffin, 5.56 g, 0.139 mol) in DMF (35 ml) was added dropwise at 55 to 65°C over about 2 hours and the mixture was stirred at the same temperature for 2 hours. After cooling to room temperature, the reaction mixture was neutralized to pH 7 to 8 with 35% hydrochloric acid (3.97 g). DMF was distilled off under reduced pressure at 60 °C and the residue was dissolved in a mixed solution of water (22 ml), brine (1.5 g) and toluene (39 ml). After the pH of the solution was adjusted to 4 with 35% hydrochloric acid, the layers were separated and an aqueous layer in the bottom was further extracted with toluene (39 ml x 2). The combined toluene layers were sequentially washed with hydrochloric acid water (35% hydrochloric acid 1.2 g and water 15 g), water (15ml) and sodium bicarbonate water (sodium bicarbonate: 0.68 g and water: 15 g) and then dried and decolorized with anhydrous magnesium sulfate and activated carbon. After filtering, the filtrate was concentrated. The resulting concentrate (26.9 g) was eluted with toluene (11 ml) and n-heptane (9 ml) to obtain 15.0 g of (2R,3S)-2-(2,4-difluorophenyl)-3-methyl-2-[(1H-1,2,4-triazol-1-yl)methyl]oxirane as a crude product. The crude product was further purified by crystallization from a solvent mixture of toluene (8 ml) and heptane (8 ml) to obtain 13.4 g of pure (2R,3S)-2-(2,4-difluorophenyl)-3-methyl-2-[(1H-1,2,4-triazol-1-yl)methyl]oxirane. Yield: 46% (based on (2R)-2',4'-difluoro-2-(1'-methoxy-1'-methylethoxy)propiophenone).
¹H-NMR(400Hz,in CDCl₃)d: 1.64(3H, d, J=6Hz), 3.19(1H, q, J=6Hz), 4.43, 4.88(each 1H, d, J=15Hz), 6.70-6.80(2H, m), 6.90-7.04(1H, m), 7.81, 7.96(each 1H, s).

### Example 6: (2R,3S)-2-(2,4-difluorophenyl)-3-methyl-2-[(1H-1,2,4-triazol-1-yl)methyl]oxirane

### (a): (2R,3R)-3-(2',4'-difluorophenyl)-3,4-epoxy-2-(1'-methoxy-1'-methylethoxy)butane

Trimethylsulfonium bromide (87.2 g, 0.50 mol,1.3 eq.) was dissolved in DMSO (600 ml) solvent. Sodium hydride (60% dispersion in liquid paraffin, 18.6 g, 0.47 mol, 1.2 eq.) was added thereto in some portions over 2.5 hours at 16 to 25 °C, and the mixture was stirred until hydrogen generation stopped. Next, liquid paraffin (48.4 g) and toluene (100 ml) were sequentially poured. The solution of (2R)-2',4'-difluoro-2-(1'-methoxy-1'-methylethoxy)propiophenone (100.0 g, 0.39 mol) in toluene (200 ml) was added dropwise over 1 hour and 45 minutes and the mixture was stirred at room temperature for 1.5 hours. After the completion of the reaction, the reaction solution was poured into 700 g of ice water, the mixture was stirred and the layers were separated. The aqueous layer was further extracted twice with toluene (400 ml and 200 ml). The combined organic layers were washed twice with water (200 ml), and then concentrated under reduced pressure to obtain 156.0 g of light yellow oil. The ratio of (2R,3R) form to (2R,3S) form was 4:1 (area percentage by HPLC).

### (b): (2R,3R)-2-(2,4-difluorophenyl)-3-(1-methoxy-1-methylethoxy)-1-(1H-1,2,4-triazol-1-yl)-2-butanol

Sodium hydride (60% dispersion in liquid paraffin, 23.3 g, 0.58 mol, 3 eq.) was added to a mixed liquid of 1,2,4-triazole (60.2 g, 0.87 mol, 4.5 eq.) and DMF (200 ml) over about 2 hours at 0 to 10°C with cooling and the mixture was stirred until generation of hydrogen stopped. Next, a solution of the mixture obtained in (a) (78.0 g, 0.19 mol) in DMF (50 ml) was added thereto and then the mixture was heated at 83 to 85 °C for about 8 hours. After cooling, the reaction was finished with water (400 ml) and the reaction mixture was extracted with toluene (200 ml, 100 ml, 100 ml) three times. The combined organic layers were washed twice with water (100 ml) and then concentrated under reduced pressure to obtain 97.3 g of mixture of (2R,3R)-2-(2,4-difluorophenyl)-3-(1-methoxy-1-methylethoxy)-1-(1H-1,2,4-triazol-1-yl)-2-butanol and its stereoisomer (2R,3S) form (4:1) as oil.
¹H-NMR(400MHz, in CDCl₃)d:
(2R,3R) form:1.18(3H, dd, J=6Hz,1Hz), 1.36,1.40(each 3H, s), 2.81, 3.21(each 1H, d, J=6Hz), 3.26(3H, s), 4.09(1H, q, J=6Hz), 6.76-6.79(1H, m), 6.81-6.90(1H, m), 7.44(1H, m).
(2R,3S) form:1.15(dd, J=6Hz,1Hz), 1.30,1.32(each, s), 2.84, 3.13(each d, J=6Hz), 3.23(s), 4.00(q, J=6Hz),6.76-6.79(m), 6.81-6.90(m), 7.44(m).

### (c): (2R,3R)-2-(2,4-difluorophenyl)-1-(1H-1,2,4-triazol-1-yl)-2,3-butanediol

The mixture obtained in (b) (97 g, 0.19 mol) was dissolved in toluene (200 ml) and methanol (20 ml). 5% hydrochloric acid (2 ml) was added and deprotection was conducted at room temperature for 25 minutes. The mixture was neutralized with sodium bicarbonate (2.5 g) and then the reaction mass was concentrated to obtain a mixture of (2R,3R)-2-(2,4-difluorophenyl)-1-(1H-1,2,4-triazol-1-yl)-2,3-butanediol and its stereoisomer (2R,3S) form (4:1).

### (d): (2R,3S)-2-(2,4-difluorophenyl)-3-methyl-2-[(1H-1,2,4-triazol-1-yl)methyl]oxirane

Toluene (100 ml), THF (200 ml) and triethylamine (29.4 g, 0.29 mol, 1.5 eq.) were poured into the mixture obtained in (c) and methanesulfonyl chloride (26.7 g, 0.23 mol, 1.1 eq.) was slowly added dropwise at 10 to 20 °C. After stirring for 30 minutes, the reaction mass was cooled to 0 to 10 °C, 15% aqueous sodium hydroxide solution (155 g, 0.58 mol, 3 eq.) was added thereto and the mixture was stirred for 1 hours. The reaction mass was allowed to stand, the layers were separated and the aqueous layer was extracted twice with toluene (100 ml). 5% brine was added to the combined organic layers, the mixture was neutralized to pH to 7 with 35% hydrochloric acid and the layers were separated. The organic layer was washed with 5% brine (100 ml). Anhydrous magnesium sulfate and activated carbon were added, the mixture was filtered and then the filtrate was concentrated under reduced pressure. Toluene (23 ml) was added thereto and the product was crystallized to obtain (2R,3S)-2-(2,4-difluorophenyl)-3-methyl-2-[(1H-1,2,4-triazol-1-yl)methyl]oxirane as white solid. Yield: 23.7 g, 48.7% (based on (2R)-2',4'-difluoro-2-(1'-methoxy-1'-methylethoxy)propiophenone).

### Example 7: (2R,3S)-2-(2,4-difluorophenyl)-3-methyl-2-[(1H-1,2,4-triazol-1-yl)methyl]oxirane

### (a): (2R,3R)-2-(2,4-difluorophenyl)-3-(1-methoxy-1-methylethoxy)-1-(1H-1,2,4-triazol-1-yl)-2-butanol

The mixed liquid of 1,2,4-triazole (40.2 g, 0.58 mol, 3.0 eq.), potassium carbonate (77.6 g, 0.56 mol, 2.9 eq.) and DMF (200 ml) was heated to 85°C. A solution of the mixture of (2R,3R)-3-(2',4'-difluorophenyl)-3,4-epoxy-2-(1'-methoxy-1'-methylethoxy)butane and its stereoisomer (2R,3S) form obtained in Example 6(a) (78.0 g, 0.19 mol) in DMF (50 ml) was added dropwise tehreto over 50 minutes. After reacting for about four and half hours at 85 to 90°C, the reaction mixture was poured into a mixed solution of water (500 ml) and toluene (200 ml) and the layers were separated. The aqueous layer was further extracted twice with toluene (100 ml). The combined organic layers were washed twice with water (100 ml) to obtain a solution of a mixture of (2R,3R)-2-(2,4-difluorophenyl)-3-(1-methoxy-1-methylethoxy)-1-(1H-1,2,4-triazol-1-yl)-2-butanol and its stereoisomer (2R,3S) form in toluene.

### (b): (2R,3R)-2-(2,4-difluorophenyl)-1-(1H-1,2,4-triazol-1-yl)-2,3-butanediol

Methanol (20 ml) and 5% hydrochloric acid (2 ml) were added to the toluene solution obtained in (a) and the mixture was stirred for about 1 hour at 19 °C. The mixture was neutralized with 15% aqueous sodium hydroxide solution (0.67 g) and the reaction mixture was concentrated under reduced pressure to about half volume to obtain a solution of mixture of (2R,3R)-2-(2,4-difluorophenyl)-1-(1H-1,2,4-triazol-1-yl)-2,3-butanediol and its stereoisomer (2R,3S) form in toluene (about 260 g).

### (c): (2R,3S)-2-(2,4-difluorophenyl)-3-methyl-2-[(1H-1,2,4-triazol-1-yl)methyl]oxirane

THF (100 ml) and triethylamine (29.4 g, 0.29 mol, 1.5 eq.) were poured into the toluene solution obtained in (b) and then methanesulfonyl chloride (26.7 g, 0.23 mol, 1.1 eq.) was added dropwise over 25 minutes at 10 to 20°C. After stirring for 20 minutes, disappearance of the raw material in the reaction solution was confirmed. Thereafter, the reaction mass was cooled to 10°C, 15% aqueous sodium hydroxide solution (155 g, 0.58 mol, 3 eq.) was added dropwise over 20 minutes and then the mixture was stirred for 40 minutes. The reaction mass was allowed to stand, the layers were separated and the aqueous layer was extracted twice with toluene (100 ml and 50 ml). 5% Brine 100ml was added to the combined organic layers, the mixture was neutralized to pH to 7 with 35% hydrochloric acid (about 27.9 g) and the layers were separated. The organic layer was washed with 5% brine (100 ml), dried over anhydrous magnesium sulfate and activated carbon and decolorized. After filtering, the filtrate was concentrated under reduced pressure, toluene (29 ml) was added thereto and the product was crystallized. 25.6 g of (2R,3S)-2-(2,4-difluorophenyl)-3-methyl-2-[(1H-1,2,4-triazol-1-yl)methyl]oxirane was obtained as white solid. Yield: 52.6%.

The compound (I) in which a protective group for a hydroxyl group is (1-methoxy-1-methyl)ethyl group can be crystallized at normal temperature. The compound (I) is a single substance and therefore, it is possible to purify by recrystallization, whereas the conventional compound (1) protected with 2-tetrahydropyranyl group is a diastereomer mixture. Also, when the compound (I) protected with a (1-methoxy-1-methyl)ethyl group was subjected to epoxidation reaction, it is possible to accomplish the same stereo-selectivity as using a pervious compound (1).

Consequently, it becomes possible to use a material having high purity and to produce an epoxytriazole compound (VII) under proper quality control in accordance with GMP more advantageously than the prior art.

## Claims

1. A compound of the formula (I); 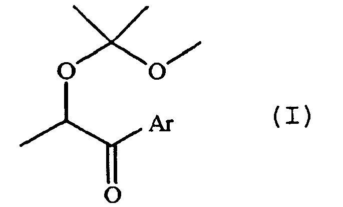 wherein Ar represents difluorophenyl group.

2. The compound according to Claim 1, wherein Ar is 2,4-difluorophenyl group.

3. The compound according to Claim 1, which has a steric configuration of the formula ( Ia ); 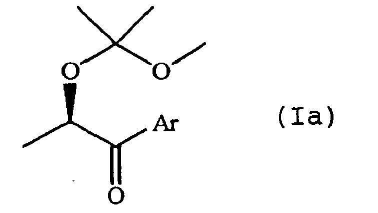 wherein Ar has the same meaning as defined above.

4. A compound of the formula ( II); 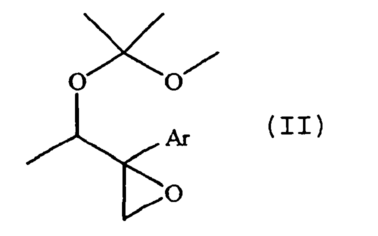 wherein Ar represents difluorophenyl group.

5. The compound according to Claim 4, wherein Ar is 2,4-difluorophenyl group.

6. The compound according to Claim 4, which has a steric configuration of the formula ( IIa ); 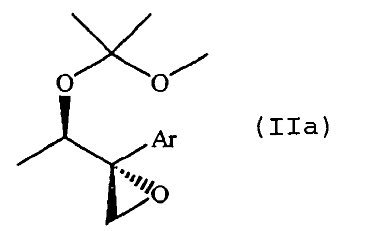 wherein Ar has the same meaning as defined above.

7. A compound of the formula (III) or a salt thereof; 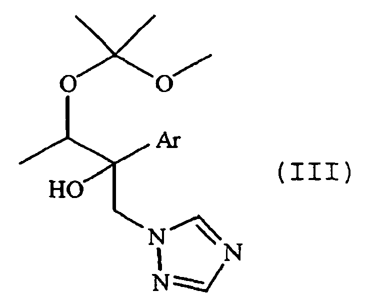 wherein Ar has the same meaning as defined above.

8. The compound or a salt thereof according to Claim 7, wherein Ar is 2,4-difluorophenyl group.

9. The compound or a salt thereof according to Claim 7, which has a steric configuration of the formula ( IIIa ); 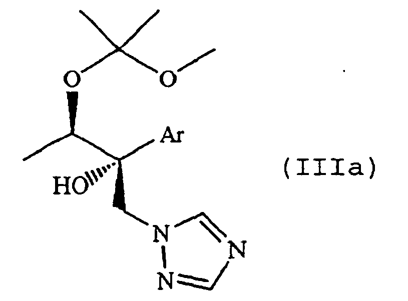 wherein Ar has the same meaning as defined above.

10. A process for producing a compound of the formula (VII) or a salt thereof; 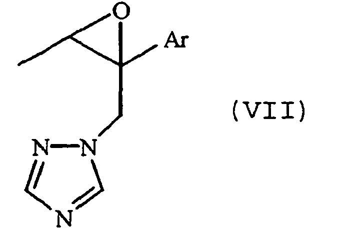 wherein Ar represents difluorophenyl group,
which comprises the following Steps (a), (b), (c) and (d).
Step (a): a step for epoxydizing a compound of the formula ( I ); 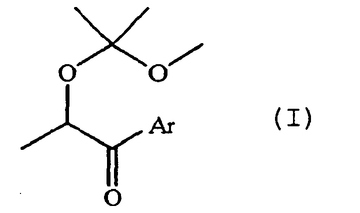 wherein Ar has the same meaning as defined above,
to obtain a compound of the formula (II); 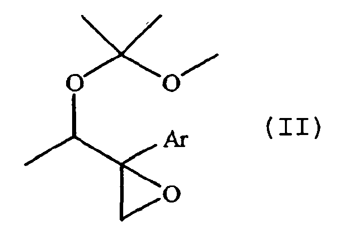 wherein Ar has the same meaning as defined above
Step (b): a step for deprotecting the compound of the formula (II) to obtain a compound of the formula (IV); 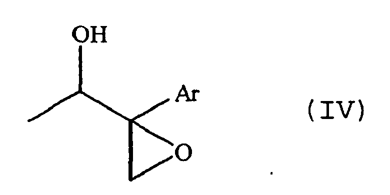 wherein Ar has the same meaning as defined above Step (c): a step for reacting the compound of the formula (IV) with a compound of the formula (V);
RSO₂X (V)
wherein R represents an alkyl group having 1 to 12 carbon atoms, phenyl group or tolyl group, and X represents halogen atom,
to obtain a compound of the formula (VI); 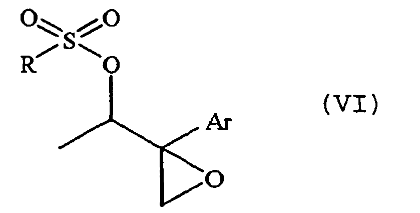 wherein R and X have the same meanings as defined above
Step (d): a step for reacting the compound of the formula (VI) with 1,2,4-triazole to obtain the compound of the formula (VII)

11. A process for producing a compound of the formula (VII) or a salt thereof; 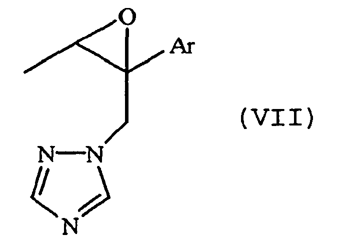 wherein Ar represents difluorophenyl group,
which comprises the following Steps (a), (e), (f), (g) and (h). Step (a): a step for epoxydizing a compound of the formula ( I ); 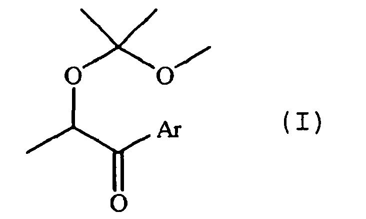 wherein Ar has the same meaning as defined above,
to obtain a compound of the formula (II); 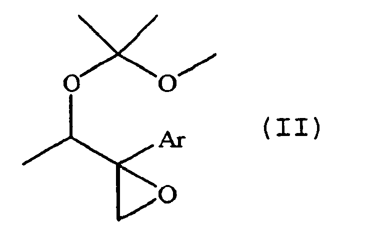 wherein Ar has the same meaning as defined above
Step (e): a step for reacting the compound of the formula (II) with 1,2,4-triazole to obtain a compound of the formula (III) or a salt thereof; 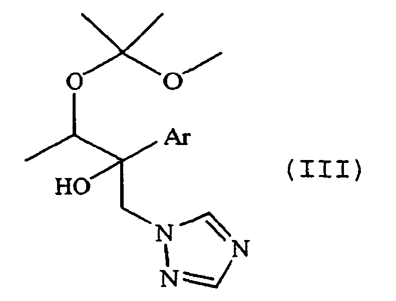 wherein Ar has the same meaning as defined above
Step (f): a step for deprotecting the compound of the formula (III) or the salt thereof to obtain a compound of the formula (VIII) or a salt thereof; 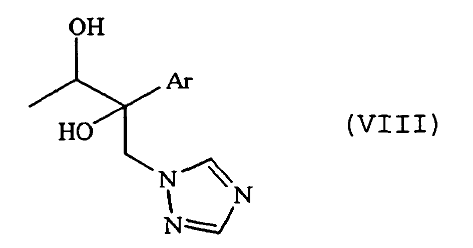 wherein Ar has the same meaning as defined above
Step (g): a step for reacting the compound of the formula (VIII) or the salt thereof with a compound of the formula (V);
RSO₂X (V)
wherein R represents an alkyl group having 1 to 12 carbon atoms, phenyl group or tolyl group, and X represents halogen atom,
to obtain a compound of the formula (IX); 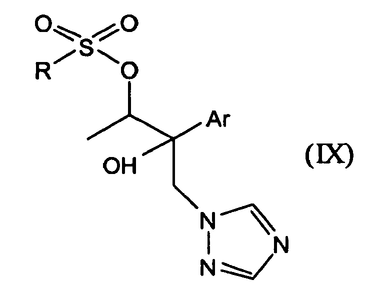 wherein R and X have the same meanings as defined above
Step (h): a step for treating the compound of the formula (IX) or a salt thereof with a base to obtain the compound of the formula (VII) or a salt thereof.

12. A process for producing a compound of the formula (I); 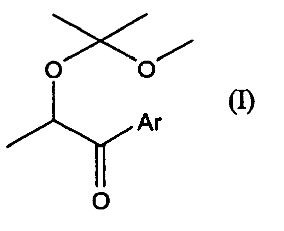 wherein Ar represents difluorophenyl group, which comprises a step for reacting a compound of the formula (XI);
ArX² (XI)
wherein Ar represents difluorophenyl group and X² represents halogen atom, with a Grignard reagent of the formula (X);
R¹MgX¹ (X)
wherein R¹ represents hydrocarbon group and X¹ represents halogen atom, to obtain an arylmagnesium halide of the formula (XII);
ArMgX¹ (XII)
wherein Ar and X¹ have the same meaning as defined above,
and a step for reacting a compound of the formula (XIII'); 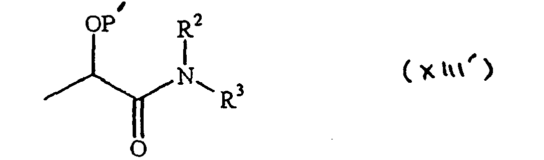 wherein P, represents a (1-methyoxy-1-methyl)ethyl group, and R² and R³ each independently represent an alkyl group having 1 to 12 carbon atoms or an alkoxy group having 1 to 8 carbon atoms or R² and R³ bond to form divalent acyclic hydrocarbon group which forms aliphatic heterocyclic ring together with an adjacent nitrogen atom, and one of -CH₂- in the divalent acyclic hydrocarbon group may be substituted by -O-, with the proviso that R² and R³ don't represent alkoxy groups having 1 to 8 carbon atoms simultaneously,
with the compound of the arylmagnesium halide of the formula (XII).

13. A process for producing a compound of the formula (I); 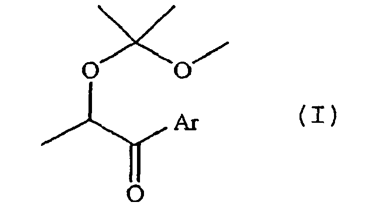 wherein Ar represents difluorophenyl group,
which comprises the following Steps (i), (j), (k) and (1).
Step (i): a step for reacting a compound of the formula (XI);
ArX² (XI)
wherein Ar represents difluorophenyl group and X² represents halogen atom, with a Grignard reagent of the formula (X);
R¹MgX¹ (X)
wherein R¹ represents hydrocarbon group and X¹ represents halogen atom, to obtain an arylmagnesium halide of the formula (XII);
ArMgX¹ (XII)
wherein Ar and X¹ have the same meaning as defined above
Step (j): a step for reacting a compound of the formula (XIII); 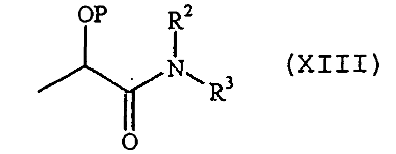 wherein P is a protecting group for a hydroxyl group, and R² and R³ each independently represent an alkyl group having 1 to 12 carbon atoms or an alkoxy group having 1 to 8 carbon atoms or R² and R³ bond to form divalent acyclic hydrocarbon group which forms aliphatic heterocyclic ring together with an adjacent nitrogen atom, and one of -CH₂- in the divalent acyclic hydrocarbon group may be substituted by -O-, with the proviso that R² and R³ don't represent alkoxy groups having 1 to 8 carbon atoms simultaneously,
with the compound of the arylmagnesium halide of the formula (XII) to obtain a compound of the formula (XIV); 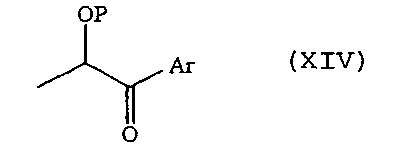 wherein P and Ar have the same meanings as defined above
Step (k): a step for deprotecting the compound of the formula (XIV) to obtain a compound of the formula (XV); 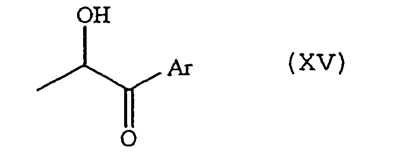 wherein Ar has the same meaning as defined above
Step (1): a step for reacting the compound of the formula (XV) with 2-methoxypropene to obtain the compound of the formula (I)

## Patentansprüche

1. Eine Verbindung der Formel (I): 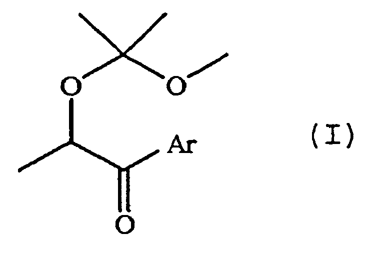 wobei Ar einen Difluorphenylrest darstellt.

2. Die Verbindung gemäß Anspruch 1, wobei Ar ein 2,4-Difluorphenylrest ist.

3. Die Verbindung gemäß Anspruch 1, welche eine sterische Konfiguration der Formel (Ia) 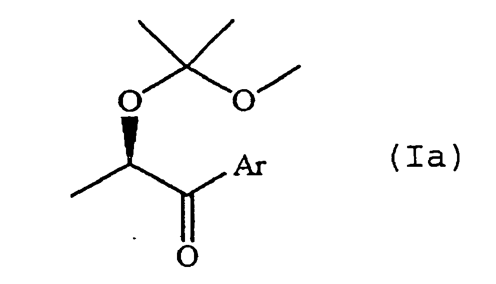 aufweist, wobei Ar die gleiche Bedeutung wie vorstehend definiert aufweist.

4. Eine Verbindung der Formel (II): 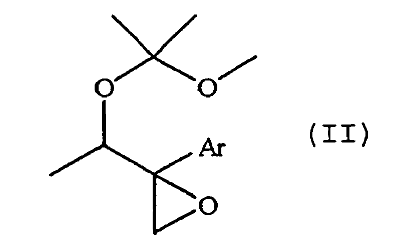 wobei Ar einen Difluorphenylrest darstellt.

5. Die Verbindung gemäß Anspruch 4, wobei Ar ein 2,4-Difluorphenylrest ist.

6. Die Verbindung gemäß Anspruch 4, welche eine sterische Konfiguration der Formel (IIa) 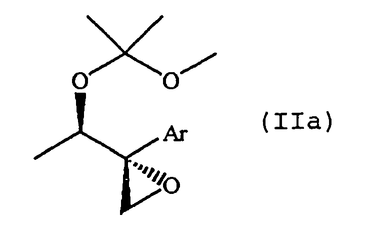 aufweist, wobei Ar die gleiche Bedeutung wie vorstehend definiert aufweist.

7. Eine Verbindung der Formel (III) oder ein Salz davon: 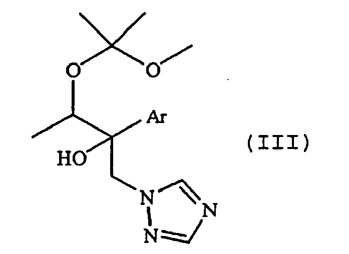 wobei Ar die gleiche Bedeutung wie vorstehend definiert aufweist.

8. Die Verbindung oder ein Salz davon gemäß Anspruch 7, wobei Ar ein 2,4-Difluorphenylrest ist.

9. Die Verbindung oder ein Salz davon gemäß Anspruch 7, welche eine sterische Konfiguration der Formel (IIIa) 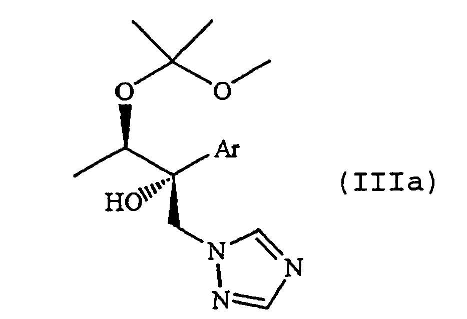 aufweist, wobei Ar die gleiche Bedeutung wie vorstehend definiert aufweist.

10. Ein Verfahren zur Herstellung einer Verbindung der Formel (VII) oder eines Salzes davon: 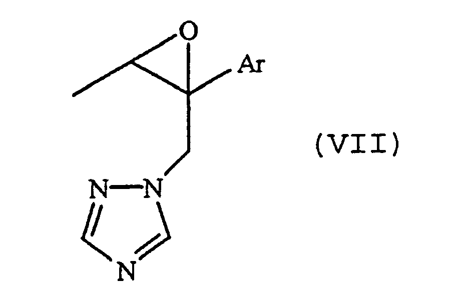 wobei Ar einen Difluorphenylrest darstellt,
welches die folgenden Schritte (a), (b), (c) und (d) umfasst:
Schritt (a): einen Schritt des Epoxidierens einer Verbindung der Formel (I): 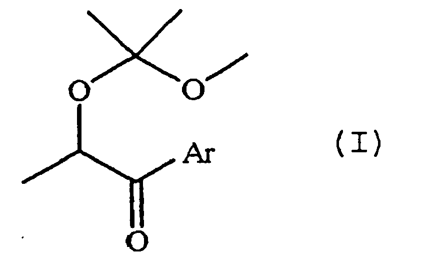 wobei Ar die gleiche Bedeutung wie vorstehend definiert aufweist,
um eine Verbindung der Formel (II) zu erhalten: 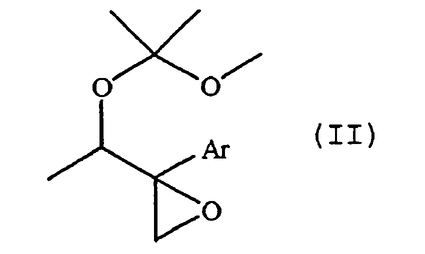 wobei Ar die gleiche Bedeutung wie vorstehend definiert aufweist;
Schritt (b): einen Schritt des Entschützens der Verbindung der Formel (II), um eine Verbindung der Formel (IV) zu erhalten: 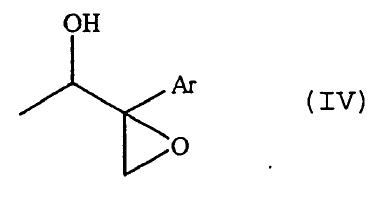 wobei Ar die gleiche Bedeutung wie vorstehend definiert aufweist;
Schritt (c): einen Schritt des Umsetzens der Verbindung der Formel (IV) mit einer Verbindung der Formel (V):
RSO₂X (V)
wobei R einen Alkylrest mit 1 bis 12 Kohlenstoffatomen, einen Phenylrest oder Tolylrest darstellt und X ein Halogenatom darstellt,
um eine Verbindung der Formel (VI) zu erhalten: 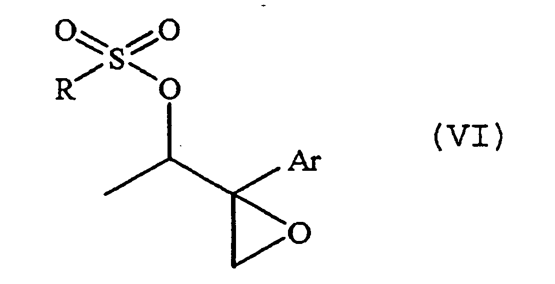 wobei R und X die gleichen Bedeutungen wie vorstehend definiert aufweisen;
Schritt (d): einen Schritt des Umsetzens der Verbindung der Formel (VI) mit 1,2,4-Triazol, um die Verbindung der Formel (VII) zu erhalten.

11. Ein Verfahren zur Herstellung einer Verbindung der Formel (VII) oder eines Salzes davon: 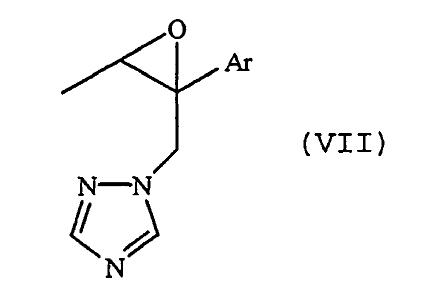 wobei Ar einen Difluorphenylrest darstellt,
welches die folgenden Schritte (a), (e), (f), (g) und (h) umfasst:
Schritt (a): einen Schritt des Epoxidierens einer Verbindung der Formel (I): 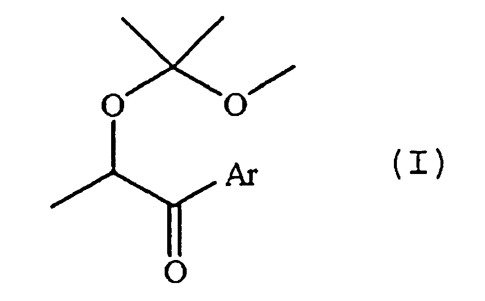 wobei Ar die gleiche Bedeutung wie vorstehend definiert aufweist,
um eine Verbindung der Formel (II) zu erhalten: 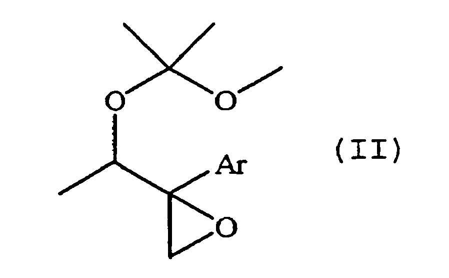 wobei Ar die gleiche Bedeutung wie vorstehend definiert aufweist;
Schritt (e): einen Schritt des Umsetzens der Verbindung der Formel (II) mit 1,2,4-Triazol, um eine Verbindung der Formel (III) oder ein Salz davon zu erhalten: 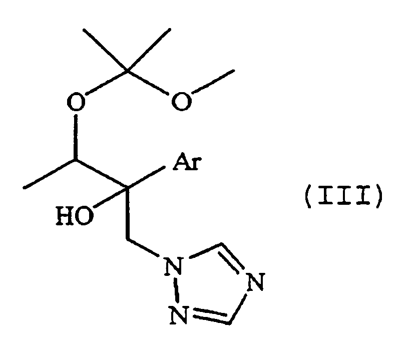 wobei Ar die gleiche Bedeutung wie vorstehend definiert aufweist;
Schritt (f): einen Schritt des Entschützens der Verbindung der Formel (III) oder des Salzes davon, um eine Verbindung der Formel (VIII) oder ein Salz davon zu erhalten: 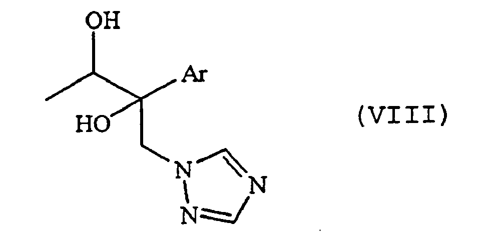 wobei Ar die gleiche Bedeutung wie vorstehend definiert aufweist;
Schritt (g): einen Schritt des Umsetzens der Verbindung der Formel (VIII) oder des Salzes davon mit einer Verbindung der Formel (V):
RSO₂X (V)
wobei R einen Alkylrest mit 1 bis 12 Kohlenstoffatomen, einen Phenylrest oder Tolylrest darstellt und X ein Halogenatom darstellt,
um eine Verbindung der Formel (IX) zu erhalten: 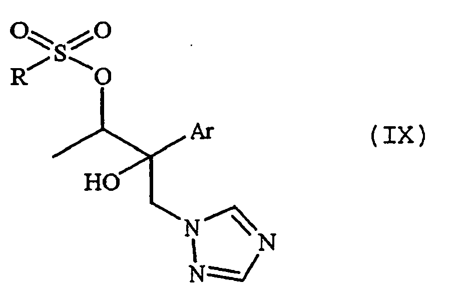 wobei R und X die gleichen Bedeutungen wie vorstehend definiert aufweisen;
Schritt (h): einen Schritt des Behandelns der Verbindung der Formel (IX) oder eines Salzes davon mit einer Base, um die Verbindung der Formel (VII) oder ein Salz davon zu erhalten.

12. Ein Verfahren zur Herstellung einer Verbindung der Formel (1): 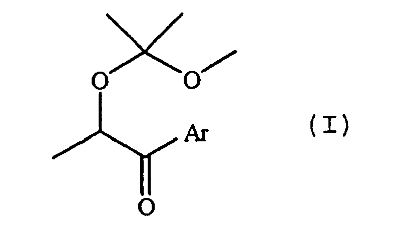 wobei Ar einen Difluorphenylrest darstellt, welches einen Schritt des Umsetzens einer Verbindung der Formel (XI):
ArX² (XI)
wobei Ar einen Difluorphenylrest darstellt und X² ein Halogenatom darstellt, mit einem Grignard-Reagenz der Formel (X):
R¹MgX¹ (X)
wobei R¹ einen Kohlenwasserstoffrest darstellt und X¹ ein Halogenatom darstellt, um ein Arylmagnesiumhalogenid der Formel (XII) zu erhalten:
ArMgX¹ (XII)
wobei Ar und X¹ die gleichen Bedeutungen wie vorstehend definiert aufweisen,
und einen Schritt des Umsetzens einer Verbindung der Formel (XIII'): 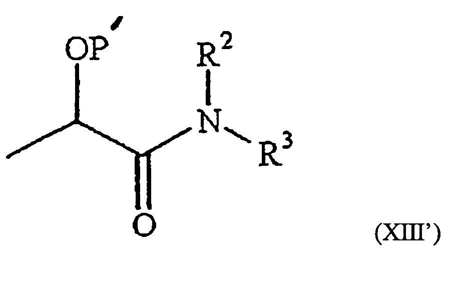 wobei P' einen (1-Methoxy-l-methyl)ethylrest darstellt und R² und R³ jeweils unabhängig einen Alkylrest mit 1 bis 12 Kohlenstoffatomen oder einen Alkoxyrest mit 1 bis 8 Kohlenstoffatomen darstellen oder sich R² und R³ verbinden, um einen zweiwertigen acyclischen Kohlenwasserstoffrest zu bilden, der zusammen mit einem benachbarten Stickstoffatom einen aliphatischen heterocyclischen Ring bildet, und einer der Reste -CH₂- im zweiwertigen acyclischen Kohlenwasserstoffrest durch -O-substituiert sein kann, mit der Maßgabe, dass R² und R³ nicht gleichzeitig Alkoxyreste mit 1 bis 8 Kohlenstoffatomen darstellen,
mit der Verbindung des Arylmagnesiumhalogenids der Formel (XII) umfasst.

13. Ein Verfahren zur Herstellung einer Verbindung der Formel (I): 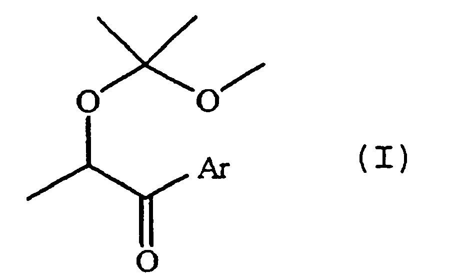 wobei Ar einen Difluorphenylrest darstellt,
welches die folgenden Schritte (i), (j), (k) und (1) umfasst:
Schritt (i): einen Schritt des Umsetzens einer Verbindung der Formel (XI):
ArX² (XI)
wobei Ar einen Difluorphenylrest darstellt und X² ein Halogenatom darstellt, mit einem Grignard-Reagenz der Formel (X):
R¹MgX¹ (X)
wobei R¹ einen Kohlenwasserstoffrest darstellt und X¹ ein Halogenatom darstellt, um ein Arylmagnesiumhalogenid der Formel (XII) zu erhalten:
ArMgX¹ (XII)
wobei Ar und X¹ die gleichen Bedeutungen wie vorstehend definiert aufweisen; Schritt (j): einen Schritt des Umsetzens einer Verbindung der Formel (XIII): 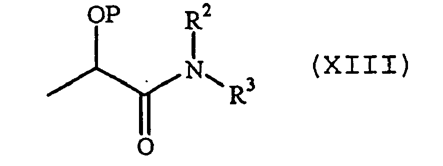 wobei P eine Schutzgruppe für einen Hydroxylrest ist und R² und R³ jeweils unabhängig einen Alkylrest mit 1 bis 12 Kohlenstoffatomen oder einen Alkoxyrest mit 1 bis 8 Kohlenstoffatomen darstellen oder sich R² und R³ verbinden, um einen zweiwertigen acyclischen Kohlenwasserstoffrest zu bilden, der zusammen mit einem benachbarten Stickstoffatom einen aliphatischen heterocyclischen Ring bildet, und einer der Reste -CH₂- im zweiwertigen acyclischen Kohlenwasserstoffrest durch -0- substituiert sein kann, mit der Maßgabe, dass R² und R³ nicht gleichzeitig Alkoxyreste mit 1 bis 8 Kohlenstoffatomen darstellen,
mit der Verbindung des Arylmagnesiumhalogenids der Formel (XII), um eine Verbindung der Formel (XIV) zu erhalten: 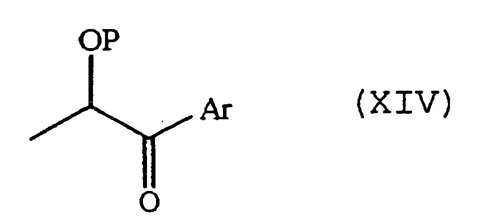 wobei P und Ar die gleichen Bedeutungen wie vorstehend definiert aufweisen;
Schritt (k): einen Schritt des Entschützens der Verbindung der Formel (XIV), um eine Verbindung der Formel (XV) zu erhalten: 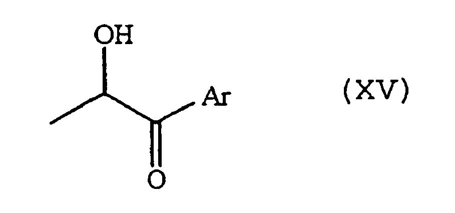 wobei Ar die gleiche Bedeutung wie vorstehend definiert aufweist;
Schritt (l): einen Schritt des Umsetzens der Verbindung der Formel (XV) mit 2-Methoxypropen, um die Verbindung der Formel (I) zu erhalten.

## Revendications

1. Composé de la formule (I) ; 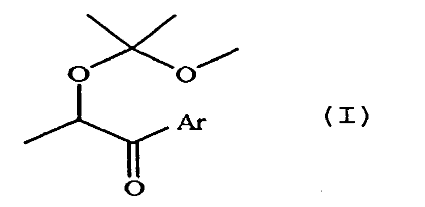 dans laquelle Ar représente un groupe difluorophényle.

2. Composé selon la revendication 1, dans lequel Ar est le groupe 2,4-difluorophényle.

3. Composé selon la revendication 1, lequel présente une configuration stérique de la formule (Ia) ; 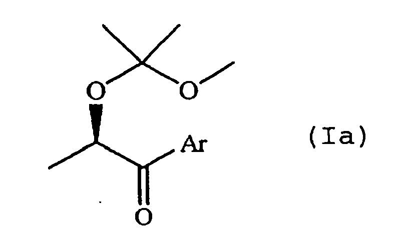 dans laquelle Ar a la même signification que définie ci-dessous.

4. Composé de la formule (II) ; 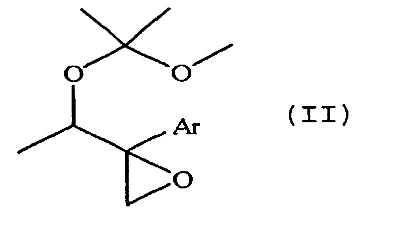 dans laquelle Ar représente un groupe difluorophényle.

5. Composé selon la revendication 4, dans lequel Ar est le groupe 2,4-difluorophényle.

6. Composé selon la revendication 4, lequel présente une configuration stérique de la formule (IIa) ; 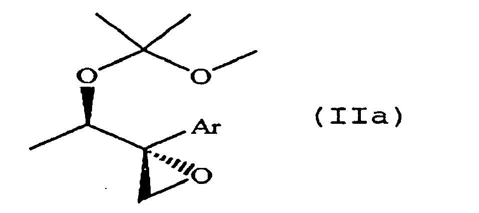 dans laquelle Ar a la même signification que définie ci-dessus.

7. Composé de la formule (III) ou sel de celui-ci ; 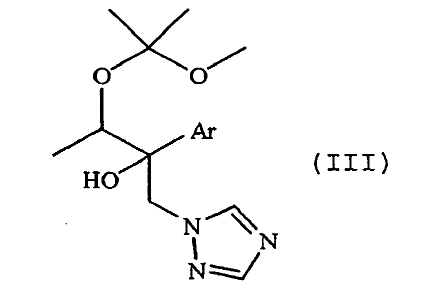 dans laquelle Ar a la même signification que définie ci-dessus.

8. Composé ou sel de celui-ci selon la revendication 7, dans lequel Ar est le groupe 2,4-difluorophényle.

9. Composé ou sel de celui-ci selon la revendication 7, lequel présente une configuration stérique de la formule (IIIa). 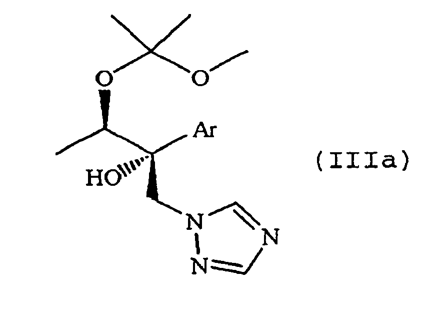 dans laquelle Ar a la même signification que définie ci-dessus.

10. Procédé de production d'un composé de la formule (VII) ou d'un sel de celui-ci ; 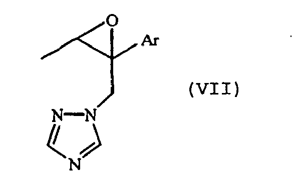 dans laquelle Ar représente le groupe difluorophényle,
lequel comprend les étapes (a), (b), (c) et (d) suivantes. Etape (a) : une étape d'époxydation d'un composé de la formule (I) ; 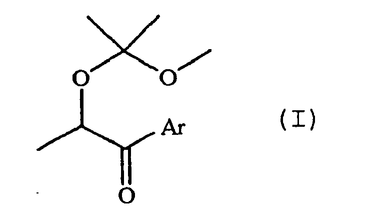 dans laquelle Ar a la même signification que définie ci-dessus, pour obtenir un composé de la formule (II) ; 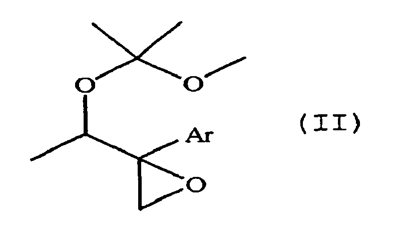 dans laquelle Ar a la même signification que définie ci-dessus
Etape (b) : une étape de déprotection du composé de la formule (II) pour obtenir un composé de la formule (IV) ; 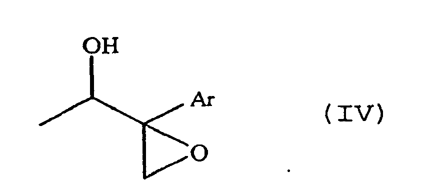 dans laquelle Ar a la même signification que définie ci-dessus
Etape (c) : une étape de réaction du composé de la formule (IV) avec un composé de la formule (V) ;
RSO₂X (V)
dans laquelle R représente un groupe alkyle ayant de 1 à 12 atomes de carbone, un groupe phényle ou un groupe tolyle, et X représente un atome d'halogène,
pour obtenir un composé de la formule (VI) ; 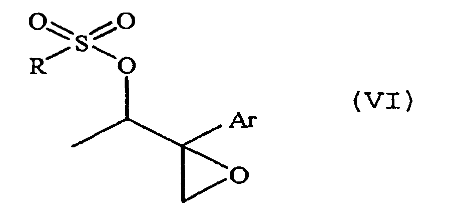 dans laquelle R et X ont les mêmes significations que définies ci-dessus
Etape (d) : une étape de réaction du composé de la formule (VI) avec du 1,2,4-triazole pour obtenir le composé de la formule (VII).

11. Procédé de production d'un composé de la formule (VII) ou d'un sel de celui-ci ; 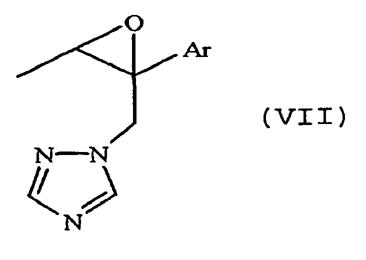 dans laquelle Ar représente un groupe difluorophényle,
lequel comprend les étapes (a), (e), (f), (g) et (h) suivantes. Etape (a) : une étape d'époxydation d'un composé de la formule (I) ; 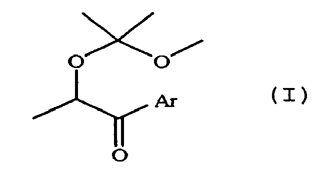 dans laquelle Ar a la même signification que définie ci-dessus, pour obtenir un composé de la formule (II) ; 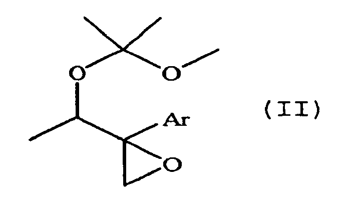 dans laquelle Ar a la même signification que définie ci-dessus
Etape (e) : une étape de réaction du composé de la formule (II) avec du 1,2,4-triazole pour obtenir un composé de la formule (III) ou un sel de celui-ci ; 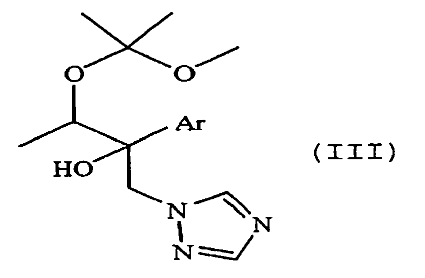 dans laquelle Ar a la même signification que définie ci-dessus
Etape (f) : une étape de déprotection du composé de la formule (III) ou du sel de celui-ci pour obtenir un composé de la formule (VIII) ou un sel de celui-ci ; 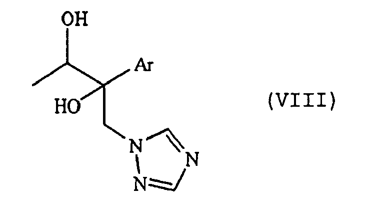 dans laquelle Ar a la même signification que définie ci-dessus
Etape (g) : une étape de réaction du composé de la formule (VIII) ou du sel de celui-ci avec un composé de la formule (V) ;
RSO₂X (V)
dans laquelle R représente un groupe alkyle ayant de 1 à 12 atomes de carbone, un groupe phényle ou un groupe tolyle, et X représente un atome d'halogène,
pour obtenir un composé de la formule (IX) ; 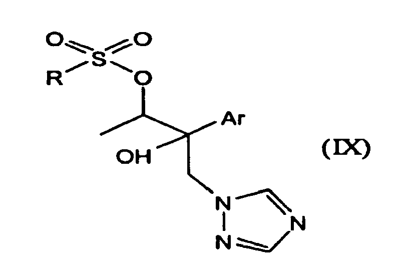 dans laquelle R et X ont les mêmes significations que définies ci-dessus
Etape (h) : une étape de traitement du composé de la formule (IX) ou d'un sel de celui-ci avec une base pour obtenir le composé de la formule (VII) ou un sel de celui-ci.

12. Procédé de production d'un composé de la formule (I) ; 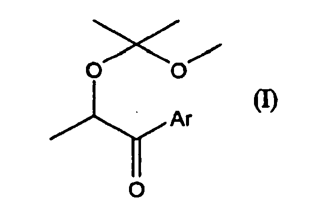 dans laquelle Ar représente un groupe difluorophényle, lequel comprend une étape de réaction d'un composé de la formule (XI) ;
ArX² (XI)
dans laquelle Ar représente un groupe difluorophényle et X² représente un atome d'halogène, avec un réactif de Grignard de la formule (X) ;
R¹MgX¹ (X)
dans laquelle R¹ représente un groupe hydrocarboné et X¹ représente un atome d'halogène, pour obtenir un halogénure d'aryle magnésium de la formule (XII) ;
ArMgX¹ (XII)
dans laquelle Ar et X¹ ont les mêmes significations que définies ci-dessus,
et une étape de réaction d'un composé de la formule (XIII') ; 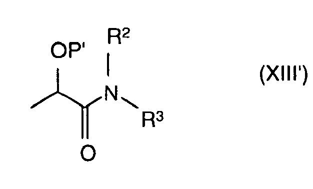 dans laquelle P' représente un groupe (1-méthoxy-1-méthyl)éthyle et R² et R³ représentent chacun indépendamment un groupe alkyle ayant de 1 à 12 atomes de carbone, un groupe alcoxy ayant de 1 à 8 atomes de carbone, ou R² et R³ se lient pour former un groupe hydrocarboné acyclique divalent qui forme un noyau hétérocyclique aliphatique avec un atome d'azote adjacent, et un des -CH₂- dans le groupe hydrocarboné acyclique divalent peut être substitué par -O-, à condition que R² et R³ ne représentent simultanément pas des groupes alcoxy ayant de 1 à 8 atomes de carbone,
avec le composé de l'halogénure d'arylmagnésium de la formule (XII).

13. Procédé de production d'un composé de la formule (I) ; 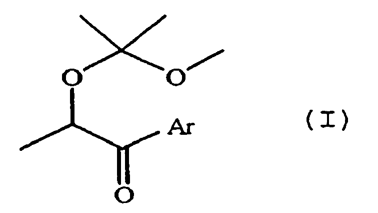 dans laquelle Ar représente un groupe difluorophényle,
lequel comprend les étapes (i), (j), (k) et (1) suivantes.
Etape (i) : une étape de réaction d'un composé de la formule (XI) ;
ArX² (XI)
dans laquelle Ar représente un groupe difluorophényle et X² représente un atome d'halogène,
avec un réactif de Grignard de la formule (X) ;
R¹MgX¹ (X)
dans laquelle R¹ représente un groupe hydrocarboné et X¹ représente un atome d'halogène,
pour obtenir un halogénure d'arylmagnésium de la formule (XII) ;
ArMgX¹ (XII)
dans laquelle Ar et X¹ ont la même signification que définie ci-dessus.
Etape (j) : une étape de réaction d'un composé de la formule (XIII) ; 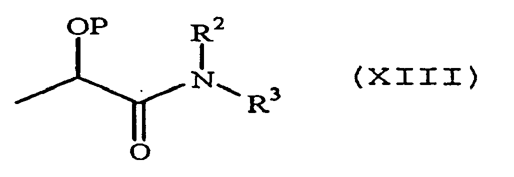 dans laquelle P est un groupe protecteur pour un groupe hydroxyle, et R² et R³ représentent chacun indépendamment un groupe alkyle ayant de 1 à 12 atomes de carbone ou un groupe alcoxy ayant de 1 à 8 atomes de carbone ou R² et R³ se lient pour former un groupe hydrocarboné acyclique divalent qui forme un noyau hétérocyclique aliphatique avec un atome d'azote adjacent, et un des -CH₂- dans le groupe hydrocarboné acyclique divalent peut être substitué par -O-, à condition que R² et R³ ne représentent pas simultanément des groupes alcoxy ayant de 1 à 8 atomes de carbone,
avec le composé de l'halogénure d'arylmagnésium de la formule (XII) pour obtenir un composé de la formule (XIV) ; 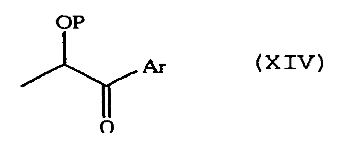 dans laquelle P et Ar ont les mêmes significations que définies ci-dessus.
Etape (k) : une étape de déprotection du composé de la formule (XIV) pour obtenir un composé de la formule (XV) ; 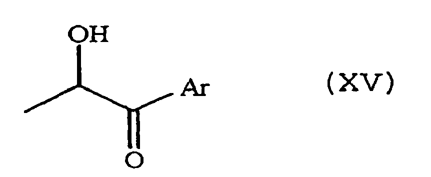 dans laquelle Ar a la même signification que définie ci-dessus
Etape (l) : une étape de réaction du composé de la formule (XV) avec du 2-méthoxypropène pour obtenir le composé de la formule (I).
